(19) Europäisches Patentamt
European Patent Office
Office européen des brevets

(11) EP 2 384 341 B1

(12) **EUROPEAN PATENT SPECIFICATION**

(45) Date of publication and mention of the grant of the patent:
**31.10.2012 Bulletin 2012/44**

(21) Application number: **09796709.5**

(22) Date of filing: **17.12.2009**

(51) Int Cl.:
***C08F 2/44*** *(2006.01)* ***A61Q 5/06*** *(2006.01)*
***A61K 8/81*** *(2006.01)*

(86) International application number:
**PCT/EP2009/067420**

(87) International publication number:
**WO 2010/076234 (08.07.2010 Gazette 2010/27)**

(54) **STYLING COPOLYMERS, STYLING COMPOSITIONS AND A PROCESS FOR MAKING THEM**

FORMGESTALTUNGSPOLYMERE, FORMGESTALTUNGSZUSAMMENSETZUNGEN UND HERSTELLUNGSVERFAHREN DAFÜR

COPOLYMÈRES POUR LE COIFFAGE, COMPOSITIONS DE COIFFAGE ET LEUR PROCÉDÉ DE FABRICATION

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **05.01.2009 EP 09150073**

(43) Date of publication of application:
**09.11.2011 Bulletin 2011/45**

(73) Proprietor: **BASF SE**
**67056 Ludwigshafen (DE)**

(72) Inventors:
- **MERTOGLU, Murat**
  **67059 Ludwigshafen (DE)**
- **RUDECK, Jana**
  **67063 Ludwigshafen (DE)**
- **LAUBENDER, Matthias**
  **67105 Schifferstadt (DE)**
- **MERZ, Ute**
  **67112 Mutterstadt (DE)**
- **SCHRÖDER, Jens**
  **67227 Frankenthal (DE)**

(74) Representative: **Reinhardt, Jürgen et al**
**BASF Personal Care and Nutrition GmbH**
**Postfach 13 01 64**
**40551 Düsseldorf (DE)**

(56) References cited:
**GB-A- 1 456 865**
**US-A- 5 280 054**
**US-A1- 2008 020 004**
**US-A1- 2008 227 871**
**US-A1- 2008 247 975**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 384 341 B1

Printed by Jouve, 75001 PARIS (FR)

## Description

The invention relates to a styling copolymer comprising colloidal silica, a styling composition of said copolymer a process for making it and its use for hair styling purposes.

Several different water soluble or water dispersible copolymers are known to be used in styling compositions such as creams, body lotions, hairsprays, hairgels, conditioners, shampoos and styling fumes. Styling copolymers on one hand have to have a certain rigidity in order to maintain the shape of skin or dressed hair. However, on the other hand, such polymers shall not be too brittle as this would lead to a kind of precipitation behavior of the styling polymer when the treated skin or hair gets dry. Different attempts are shown in the prior art to properly tailor copolymers or styling compositions such that they meet the above-mentioned requirements.

WO 2005/082 322 A1 provides an approach using at least two components, one of which is a copolymer viz. a ter polymer comprising vinylpyrrolidone, metacrylamide and vinylimidazole. The other one is an excipient, an additive and/or an active ingredient. One such additive is a silicon compound used in an amount of 0.01 to 15 w %, said silicon compound being selected from volatile or nonvolatile and soluble or insoluble silicon compounds. Said silicon compound may have a high molecular weight and a viscosity ranging from 1000 to 2 Mio. cSt when measured at 25 °C.

Compounds having the shape of particles can also be used as additives. Said granular compounds in particular comprise silica and alkali-salts, which are preferably dispersed in a solvent and precipitate in solid form once the solvent is evaporated. A stable dispersion of said additives is obtained by supplementing them with a flow limit into the reaction mixture to be prepared.

Also the examples of WO 2005/082 322 A1 reflect the concomitant use of a copolymer e.g. Luviset® Clear and another compound in order to obtain liquid gels, spraying gels, variable mousses or hairsprays.

This means that additional substances like additives are required for fine tuning the physicochemical properties of the copolymer used. These additional substances are neither physically attached nor chemically connected to the copolymer. They are only finely dispersed, i.e. they have to be properly mixed with the copolymer in order to provide a more or less homogeneous and stable dispersion. This is laborious, time-consuming and expensive. Demixing can occur at any time for instance due to temperature changes which will yield copolymers having properties alternating for instance with temperature, as additional substances are not always in the same distance from the copolymer, thus inhibiting any constant or proper interaction with the copolymer. A highly stable headdress or a good skin treatment however requires a composition comprising a copolymer showing properties, not varying with climatic conditions. This cannot be readily achieved with such kind of copolymers.

It is therefore an object of the present invention to overcome the afore-mentioned drawbacks of the prior art and to provide an improved copolymer as well as a simpler hairstyling composition which both enable the user to easily apply it to the hair and to the naked in particular haired skin. Both, the composition and the copolymer, should enhance the sticking properties of wet hair but should not remain sticky in dry hair. They should provide the capacity of maintaining the hairstyle once fashioned and especially should increase curl retention even under harsh climatic conditions, which contributes to increase hair fixative and stiffening properties. However, the copolymers and compositions of the invention should also be very subtle and smooth in their behavior in order to avoid precipitation from the headdress formed. They should be well supported by the moistened skin especially the scalp and soluble or dispersible in water. Said copolymers and compositions should be easily obtained in high quality, purity and variable quantities by a cheap production process, said process being another topic of the invention.

Main features of the invention can be gathered from the claims 1, 13, 14 and 26, whereas further embodiments are disclosed in Claims 2 to 12 and 15 to 25.

A copolymer of the invention comprises colloidal silica and a copolymerized monomeric portion of at least one water-soluble monoolefinic monomer A and at least one monoolefinic silane monomer B, the weight portion of said monomer B being less than 1 % of the total weight of the monomeric portion.

Such copolymers of the invention increase hair fixative properties. This is to say that said copolymers enable styled wet hair to maintain its shape even when dried as measured by the stiffening effect, the sensory stickiness and the stickiness according to Kempf. Even tremendously changing climatic conditions are not prone to disarrange or even to destroy the hairstyle obtained by means of the polymers of the invention. In particular curl retention and stiffening effect are maintained for quite long times i.e. several days depending on the climatic conditions. Another advantage of said copolymers or hairstyling compositions of the invention is to make the wet hair more sticky yielding the hairstyle to be more stable and being prepared in a faster way. However, the combination of monoolefinic monomer A, monoolefinic silane monomer B in the given concentration and colloidal silica yields a copolymer which still remains soluble or at least dispersible in water and therefore can be easily removed by simply washing the hair with a mild aqueous detergent solution. Said solubility or dispersibility is conserved even when the copolymer of the invention gets dry.

The presence of colloidal silica particles in the copolymers and in the hair styling compositions of the invention excels the sensoric stiffness of the headdress and preserves the curl retention of the treated hair. It provides stickiness to the wet hair and during the drying process thereof, said stickiness rendering hairstyling easier. The aspect of the hair

once dry does barely differentiate from untreated hair as sticking properties of the copolymer or the hairstyling composition of the invention disappear once drying of the headdress is finished.

The advantageous properties of the copolymers of the invention and of the corresponding hairstyling compositions can be obtained if the colloidal silica particles are added prior or during the polymerization of monomer A in the presence of the vinylic reactive silane monomer B. It was found that preparing surface treated silica particles by means of reacting them with a representative of monomer B and once this reaction completely finished reacting said silanized particles with a water-soluble monoolefinic monomer A of the invention does not give copolymers having the properties of the copolymers and hairstyling compositions of the invention. This is not yet properly understood but may be due to a different shielding of the silica particle with monomer B when realized in a separate step compared to the simultaneous or overlapping treatment of silica particles with monomer A and monomer B as disclosed for the invention.

The monomeric portion comprises between 30 and 99.9 w % of monomer A, preferably between 60 and 99.2 w % thereof and in a highly preferred embodiment between 80 and 99.1 w %. Such a high quantity of monomer A leads to copolymers of the invention which can be readily obtained by radical polymerization in solution, which is easy to be conducted and cost saving. No complicated work up is required and the copolymer of the invention obtained with these quantities of monomer A is soluble in water or at least water-dispersible.

Prior to introducing the different compounds and compound mixtures as well as characteristics to be understood under monomer A, various definitions are made.

The term w % or % by weight or percent by weight as used in the description and in the claims denotes for a weight amount of a compound or a mixture in relation to the total weight amount of the same or another compound or mixture.

The term alkyl comprises single-chain and branched alkyl-groups. Appropriate alkyl-groups having short carbon-chains are linear or branched alkyl-groups of the type $C_1$-$C_8$-alkyl, preferentially $C_1$-$C_6$-alkyl, and in a still more preferred embodiment $C_1$-$C_4$-alkyl. Candidates comprised within said group are methyl, ethyl, propyl, isopropyl, n-butyl, 2-butyl, sec.-butyl, tert.-butyl, n-pentyl, 2-pentyl, 2-methylbutyl, 3-methylbutyl, 1.2-dimethylpropyl, 1.1-dimethylpropyl, 2.2-dimethylpropyl, 1-ethylpropyl, n-hexyl, 2-hexyl, 2-methylpentyl, 3-methylpentyl, 4-methylpentyl, 1.2-dimethylbutyl, 1.3-dimethylbutyl, 2.3-dimethylbutyl, 1.1-dimethylbutyl, 2.2-dimethylbutyl, 3.3-dimethylbutyl, 1.1.2-trimethylpropyl, 1.2.2-trimethylpropyl, 1-ethylbutyl, 2-ethylbutyl, 1-ethyl-2-methylpropyl, n-heptyl, 2-heptyl, 3-heptyl, 2-ethylpentyl, 1-propylbutyl, or octyl.

Suitable alkyl-groups having longer chains comprise $C_8$-$C_{30}$-alkyl and $C_8$-$C_{30}$-alkenyl moieties of linear or branched nature representative examples of which are n-hexyl(en), n-heptyl(en), n-oktyl(en), n-nonyl(en), n-decyl(en), n-undecyl(en), n-dodecyl(en), n-tridecyl(en), n-tetradecyl(en), n-pentadecyl(en), n-hexadecyl(en), n-heptadecyl(en), n-oktadecyl(en) and n-nonadecyl(en), wherein the phrase (en) is optional and means that one or more double-bonds can be located at any position of the aforementioned longer chain alkyl moieties.

Alkyl also means a hydrocarbon backbone, which comprises 1 to 5 substituents, said substituents being selected from the group of cycloalkyl, aryl, hetaryl, acyl, COOH, carboxylate, unsubstituted amide, N-mono-($C_1$-$C_{16}$)-alkylamide, N- di-($C_1$-$C_{16}$)-alkylamide, -$SO_3$H, sulfonate and alkylaminocarbonyl. Said substituents may not only be connected to the hydrocarbon backbone but may also form an alkyl-group as such.

The term cycloalkyl comprises hydrocarbon ring-structures having from 3 to 8 carbon-atoms as for instance cyclopentyl, cyclohexyl, cycloheptyl or cyclooctyl. Cycloalkyl-groups like cyclopentyl, cyclohexyl or cycloheptyl are free of substituents, preferably have 1,2 or 3 substituents and in a even more preferred embodiment comprise only 1 substituent connected to the cycloalkyl ring.

Alkyl-groups also comprise heterocycloalkyl rings which is to say heterocyclic rings with 4 to 7 ring atoms and preferably 5 to 6 ring atoms, 1 or 2 of said ring atoms being different from C and for instance are selected from the group of O, N, and/or S. Examples of such heterocycloaliphatic groups are pyrrolidinyl, piperidinyl, 2,2,6-tetramethylpiperidinyl, imidazolidinyl, pyrazolidinyl, oxazolidinyl, morpholinyl, thiazolidinyl, isothiazolidinyl, isoxazolidinyl, piperazinyl, tetrahydrothiophenyl, tetrahydrofuranyl, tetrahydropyranyl, N-pyrrolidonyl, N-caprolactamyl and dioxanyl. Among these radicals, N-pyrrolidonyl was found to be very well adapted to cosmetic purposes.

As previously mentioned the term alkyl also comprises different aryl moieties. Said aryl moieties include phenyl, benzyl, toluyl, xylyl, mesityl, naphthyl, fluorenyl, antracenyl, phenantrenyl or naphtacenyl moieties which are either substituted or unsubstituted. Most preferred aryl moieties are phenyl and naphthyl.

The hetaryl moieties which make also part of the alkyl term comprise unsubstituted and substituted heterocycloaromatic groups such as pyridyl, chinolinyl, acridinyl, pyridazinyl, pyrimidinyl, pyrazinyl, pyrrolyl, imidazolyl, pyrazolyl, indolyl, purinyl, indazolyl, benzotriazolyl, 1,2,3-triazolyl and carbazolyl.

Highly preferred hetaryl moieties of the invention are imidazolyl moieties as they show a certain rigidity on one hand and on the other hand are not so large in size as to hamper the flexibility of the copolymers of the invention.

The water-soluble monoolefinic monomer A and the monoolefinic silane monomer B together make the monomeric portion of the invention and their respective weight percentage values add to 100. Different types of monomers are suitable as water-soluble monoolefinic monomer A, also called monomer A as shown in figure 1.

Fig. 1

(1) 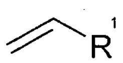 (2) 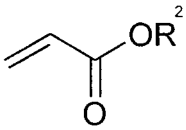 (4) 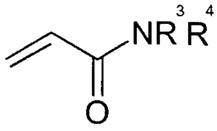

(3) 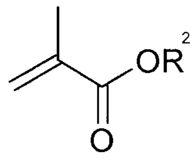 (5) 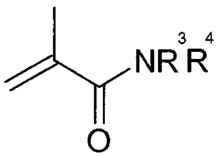

$R^1$, $R^2$, $R^3$, $R^4$ = H, Alkyl

One realizes monomer A to be selected from the group consisting of a vinylic (1), an acrylic (2), a methacrylic (3), an acrylamide (4) or a methacrylamide (5) backbone with $R^1$, $R^2$, $R^3$ and $R^4$ being hydrogen or alkyl wherein alkyl corresponds to the moieties previously mentioned.

For $R^1$, lactames such as N-pyrrolidonyl, N-caprolactamyl, N-piperidonyl, further N-imidazolyl, N-pyridinyl and carboxamidyl are highly preferred, as polymers or copolymers made from monomers or monomer mixtures having said alkyl groups are physiologically acceptable and do not provide biphasic systems upon contact with water.

$R^2$, $R^3$ and $R^4$ are preferably selected from H or alkyl having an aliphatic nature in order to avoid the copolymer formed to be too rigid and thus brittle.

Monomer A of the monomeric portion can be a single entity. However better results as to both flexibility and stability of the copolymer of the invention were obtained when the monomeric portion comprises between two and five different monomers A. This gives the opportunity to adjust the copolymer's property by balancing between bulky and less bulky monomers A. In fact very good results are obtained when the monomeric portion comprises three different monomers A, two of which are bulky and one is not bulky.

It was found that monomer or monomers A contribute to form a stable copolymer of the invention if said monomer or monomers A consist of N-vinylpyrrolidone, 1-vinylimidazole and a flexible monomer. N-vinylpyrrolidone and 1-vinylimidazole are present in excess. N-vinylpyrrolidone and 1-vinylimidazole both have a bulky ring structure which however is polar thus soluble in water. This concomitant use of 1-vinylimidazol and N-vinyl-2-pyrrolidone confers the copolymer of the invention to be both sufficiently rigid and water soluble or dispersible. It thus will remain in the hair even under highly unfavourable climatic conditions but be readily washed out by rinsing it with detergent supplemented water.

In a highly preferred embodiment of the invention monomer or monomers A consist of N-vinylpyrrolidone, 1-vinylimidazole and a flexible monomer with N-vinylpyrrolidone and 1-vinylimidazole together making between 75 and 85 w % and preferably between 78 and 81 w % of the monomeric portion. This high percentage of bulky monomers leads to a more pronounced fixation of the copolymer of the invention in the hair but on the other hand does not lead to a flaking phenomenon due to the additional presence of the more flexible monomer acting like a hinge between the more cumbersome monomers N-vinylpyrrolidone and 1-vinylimidazole.

The monomeric portion thus in a preferred embodiment of the invention also comprises a flexible monomer which is selected from the group consisting of acrylates (2), methacrylates (3), acrylamides (4) and methacrylamides (5), with R2 = H, linear or branched acyclic alkyl comprising from 1 to 6 carbon atoms and R3, R4 = H, linear or branched acyclic alkyl comprising from 1 to 6 carbon atoms. Cyclic or aromatic structures are not within the residues R1, R2, R3, R4 of the flexible monomer.

If the flexible monomer within monomers A does provide very poor rigidity, this may impair the stability properties of the copolymer of the invention, provided that the flexible monomer carries only small residues R2, R3 and R4 like H, or methyl. It is therefore particularly useful to use small entities for the flexible monomer which on the other hand are sterically less prone to bond rotation. A highly adapted flexible monomer candidate thus is methacrylamide with R3, R4 = H, or methacrylic acid with R2 = H, as it is a small entity and bond rotation is restricted due to the methyl group in the alpha position and the amide or the carboxy group. The best results were obtained with methacrylamide with R3, R4 = H, as it is a suitable hinge for the bulky monomers and on the other hand retains the desired rigidity.

Monomer A comprises between 15 and 25 w % and more preferably between 19 and 21 w % of the flexible monomer. This amount is sufficient for making the copolymer of the invention sufficiently flexible to avoid flaking from the dry hair without undermining its ability to maintain the hairstyle.

In a preferred embodiment of the invention monomer A has a water solubility of greater than 100 g/l. Said high tendency to get solubilized in water confers the copolymers of the invention to get immediately washed out from wet hair or removed from moistened skin by means of a mild detergent.

Polymerizing the components of monomer A per se however would not lead to copolymers sufficiently sticky in the moist state to satisfactorily shape and maintain a headdress. Said copolymers are not able to sufficiently attach to each other and to form interlinked structures responsible for prolonged maintaining of a hair style.

The invention thus further requires monomer B the weight portion of which ranges from 0.01 to 0.99 % of the total weight of the monomeric portion, preferably from 0.1 to 0.9 % and, in a mostly preferred embodiment from 0.2 to 0.8 %. Monomer B serves as a kind of primer for the colloidal silica which is to say yields in a stronger chemical and physical interaction between colloidal silica and monomers A. Choosing its concentration to small will not give the desired primer effect, however if said monomer B concentration is to high the amount of colloidal silica and the degree of dilution in the reaction mixture during the polymerization process have to be carefully adjusted and thoroughly monitored. Otherwise, that means if a reaction mixture containing huge amounts of monomer B will not be properly diluted and adjusted, extensive crosslinking of the components of the invention (monomer A, monomer B, colloidal silica) will take place, yielding a highly viscous copolymer which cannot be easily solubilized or dispersed in aqueous solvents and formulated in hair styling compositions.

Another advantage of monomer B is to make the copolymer of the invention even more elastic, which is necessary, as colloidal silica has a strengthening effect (cf. below) which is to be compensated. Even stormy weather considerably stretching and bending the hair and thus the copolymers of the invention cannot destroy the headdress due to the persisting i.e. not fractured structure of the copolymers of the invention.

Said monomer B is a good primer when it is a vinylic reactive silane monomer. Reactive means the monomer B to be polymerized by radical polymerization. The term vinylic comprises every structure having a double bond. One realizes said monomer B to have two different sites of reactivity one of which is the radically polymerizable vinylic moiety the other one being a differently alkylated and/or alkoxylated silylgroup. Thus polymerization can take place in a way to form a copolymer having a backbone which is highly branched, leading to structures jamming each other.

The primer monomer B serves as a hinge between monomer(s) A and the colloidal silica. It arranges for the silica particles to be in an appropriate distance from monomer(s) A which is to say that it is designed such that monomer (s) A, monomer B and colloidal silica form copolymers wherein silica particles are aligned as in a pearl necklace, the silica particles being bond both covalently and by means of physical interaction.

Monomer B is required to have the general formula $R^5$-$(CH_2)_n$-$Si(R^6)_2R^7$ with $R^5$ = acryloyloxy-, methacryloyloxy-, vinyloxy-, vinyl-, n = 0 to 10, $R^6$ = -O-$(CH_2)_m$-$CH_3$ with m = 0 to 4 isopropyl-, isopropyloxy-, tert-butyl, tert-butyloxy-, sec-butyl, sec-butyloxy- and $R^7$ = $R^6$, -H, -$CH_3$, -$CH_2CH_3$, in order to provide the copolymer of the invention with the aforementioned necklace-like structure. Said structure induces the copolymers of the invention to twist which gives them a higher strength of fixation to each other compared to non-twisted, i.e. silica free copolymers. Said higher fixation strength on the other hand does not reduce the copolymer's flexibility.

Good results were obtained with $R^5$ = acryloyloxy-, methacryloyloxy-, vinyloxy- and vinyl-. Said entities can be readily reacted by radical polymerization with the aforementioned monomers, as they are quite small in size which contributes to fast and economical copolymer formation. They are connected to a linker consisting of 0 to 10 methylene groups ($(CH_2)_n$ with n = 0 to 10). If n exceeds 10, silica particles interact with silyl groups of monomer B, which are located rather far from the copolymer backbone which is to make silica particles to liberally move. If so, they can avoid to meet each other and friction between different strands of the copolymers thus formed is reduced, leading to an undesired lower strength of fixation of said copolymers.

Silyl groups of monomer B are connected to the terminal position of the linker and $R^6$ and $R^7$ comprise different alkoxy residues selected from the group of methoxy, ethoxy, propoxy, butoxy, pentoxy, isopropyl-, isopropyloxy-, tert-butyl-, tert butyloxy-, sec-butyl, sec-butyloxy-, as well as hydrogen, methyl or ethyl. Said residues $R^6$ and $R^7$ are partially hydrolyzed during the copolymerization process and contribute to make a balance of hydrophobic and hydrophilic parts of the copolymer of the invention thus giving it an appropriate ability to connect to the negatively charged hair and at the same time to get rinsed out with detergent containing water.

One preferable candidate of monomer B comprises $R^6$ to be selected from the aforementioned alkoxy groups and $R^7$ to be H, methyl or ethyl. This is to differentiate the reactivity between the moieties connected to the Si-atom which has an impact on the chemical and physical connection of silica particles to it. Especially it influences on the amount of silica particles attached to the monomeric portion, and in particular attached to monomer B during copolymerization and thus on the strength of fixation of the copolymers formed. Choosing $R^6$ and $R^7$ to be identical entities cannot give said differentiation of reactivity with two exceptions.

One is to provide as monomer B (3-methacryloyloxy)-propyl trimethoxysilane (MEMO) having the structure $H_2C=C(CH_3)CO_2(CH_2)_3Si(OCH_3)_3$ viz. a monomer comprising three methoxy groups at its silyl moiety. It was found that copolymers of the invention obtained with (MEMO) as monomer B attach an adequate amount of colloidal silica and thus retain an appropriate interpolymeric strength of fixation regardless of the three residues $R^6$ and $R^7$ being methoxy.

The second monomer B candidate providing comparable results is vinyltrimethoxysilane (VTMOS) having the structure $H_2C=CH-Si(OCH_3)_3$, also comprising three methoxy groups at its silyl moiety.

In one embodiment of the invention, monomer B is a mixture of (3-methacryloyloxy)-propyl trimethoxysilane (MEMO) and vinyltrimethoxysilane (VTMOS). The weight ratio of 3-methacryloyloxy)-propyl trimethoxysilane (MEMO) and vinyltrimethoxysilane (VTMOS) in said mixture ranges from 1 to 9 to 9 to 1.

In another important embodiment of the invention, the copolymerized monomeric portion further comprises an olefinic monomer C. Said olefinic monomer C has a water solubility of 100 g/l or less. It is not mandatory for each copolymer of the invention to comprise monomer C, but it is highly recommend for most of them. In particular it is required if monomer A has a water solubility of greater than 100 g/l.

In the embodiment disclosed in the last paragraph, monomer A, monomer B and monomer C together make the monomeric portion viz. their respective percentage values add to 100 %.

The monomer C is selected from the group of monomers having a water solubility of 100 g/l or less. Monomer C does not necessarily comprise only one monomer. In a particular embodiment it contains different monomers of the C-type, viz. monomers which have a water solubility of 100 g/l or less.

Another substantial compound for obtaining the inventive embodiment is colloidal silica. Per 100 parts by weight of monomeric portion the copolymer of the invention comprises between 0.01 and 50 parts by weight, preferably between 0.1 and 30 parts by weight, and in a still more preferred embodiment between 0.2 and 20 parts by weight of said colloidal silica

Inventive copolymers comprising colloidal silica are very elastic and do not decompose or break if treated hair is thoroughly bent or twisted. This is not only due to the backbone of the monomeric portion but also results from the amount of colloidal silica particles present. These particles at least partially attach to the copolymer in status nascendi viz. during the polymerization process and lead to intermingled copolymer fibers of the invention having the already mentioned pearl necklace like structure. Said silica particles are in part covalently bond to the polymerized monomeric portion and partially interact by means of hydrogen bonding or other physical attractive interaction.

This leads the copolymers of the invention to be readily removed from a recipient, for instance a beaker, a reactor or a flask, in form of long and thin fibers by means of a spatula or a glass rod, which however is not possible with copolymers obtained from the monomeric portion only.

Thus colloidal silica confers to the copolymer of the invention not only a flexible but also a rigid aspect, which is to say that said copolymer has a considerable inter- or intramolecular strength of fixation. Said strength of fixation is determined by means of the aforementioned spatula test. Long fibers have a high strength of fixation whereas shorter ones are considered to have small or no strength of fixation. Copolymers with a high strength of fixation maintain their long fiber shape even under harsh climatic conditions. Said strength of fixation gets conserved in the hair styling compositions comprising said copolymer and is quantified by the stiffening effect explained below. A large stiffening effect as obtained for the hair styling compositions of the invention is a prerequisite for maintaining a headdress once formed.

Per 100 parts by weight of monomeric portion one uses up to 50 parts by weight of colloidal silica. Higher amounts result in the copolymer of the invention to disintegrate in the hair once dried observed as flaking out of white particles from the hairstyle.

High weight amounts of colloidal silica particles between 30 and 50 parts by weight can only be used if the particle size is very small, i.e. ranges from 5 to 15 nm of medium particle size and well distributed as in solutions of colloidal silica. However care must be taken as to shelf life and stability of colloidal silica solutions having such small particles. They tend to aggregate which leads to larger particles and thus to a particle fall out from dried hair.

For weight amounts of colloidal silica of up to 30 parts by weight per 100 parts by weight of the monomeric portion larger particles ranging from 5 nm to 75 nm of medium particle size are to be used without observing a precipitation of particles from the styled hair.

In a highly preferred embodiment of the invention the copolymer comprises per 100 parts by weight of monomeric portion between 0.2 and 20 parts by weight of colloidal silica. Said amount of silica still provides the desired styling effect of the copolymer thus synthesized and can be easily handled in the process of copolymerization. With larger amounts mixing and stirring requires more powerful stirring means which makes the production of the copolymers of the invention more expensive.

Easier processing is also achieved by using per 100 parts by weight of monomeric portion between 0.2 and 20 parts by weight of colloidal silica said silica having a medium particle size of from 15 to 55 nm, as smaller particles require less energy for getting mixed during the polymerization process.

The lowest amount of colloidal silica used will influence the strength of fixation of the copolymer formed and thus directly correlates to the styling performance of the copolymer or the hair styling composition. If copolymers or hair styling compositions with an extensive styling performance are desired which is the case for styling gels or hair sprays, preferentially per 100 parts by weight of monomeric portion at least 0.2 parts by weight of colloidal silica are used for the copolymer to be synthesized. However, for hair conditioners or shampoos a lower styling performance is required which is to be already achieved with smaller amounts of colloidal silica making at least 0.01 or 0.1 parts by weight of

colloidal silica per 100 parts by weight of monomeric portion.

It was further detected that colloidal silica particles having a specific surface area ranging from 50 to 500 $m^2/g$, more preferably from 100 to 350 $m^2/g$, even more preferably from 200 $m^2/g$ to 300 $m^2/g$ and most preferably being 200 $m^2/g$, provide so many sites of connection with the monomeric portion, viz. with monomer B that a certain amount of it gets covalently bond to the monomeric portion. This is preferred as it renders the copolymer of the invention more stable. Colloidal silica particles having a specific surface area of 500 $m^2/g$ should be most appropriate in this regard but do not show stability over such an extended pH range as do those colloidal silica particles, having a specific surface area of 350 $m^2/g$, 300 $m^2/g$ or of 200 $m^2/g$. The use of the latter ones is thus more preferred as it leaves more options of reaction processing. Particularly suited are colloidal silica particles having a specific surface area ranging from 300 $m^2/g$ to 200 $m^2/g$ as they unify both pH-stability of a large pH-range and many sites of connection for monomer B thus giving a copolymer of the invention providing a good strength of fixation even if produced at different pH-values.

Colloidal silica having the aforementioned surface areas of 200 $m^2/g$ and 300 $m^2/g$ and to a lesser extent of 350 $m^2/g$ were also found to show low tendency to aggregate, thus having an advantageous higher shelf fife.

The colloidal silica used for preparing copolymers of the invention is a gel or a sol obtained either from different forms of solid silica or from solution-born silica. It has a concentration of solid matter ranging from 15 to 50 w %, preferably from 25 to 40 w % and in a most preferred embodiment from 30 to 40 w % based on the weight amount of the silica gel or the silica sol used.

Different types of colloidal silica were analyzed in the process of copolymer formation such as pyrolized colloidal silica, colloidal silica obtained by chemical and physical vapor deposition techniques and colloidal silica as present in silica sols. The latter is a solution-born colloidal silica. It was found that the colloidal silica having a morphological structure as found in a silica sol, viz. a colloidal silica produced from molecularly dissolved silicic acid, provided copolymers of the invention showing an increased strength of fixation. This is to be attributed to the small size of particles in a silica sol, to their very fine and homogeneous particle repartition leading a huge amount of particles to connect to the monomeric portion and largely to the fact that the sol particles once generated from silicic acid never get completely dry prior to polymerization which would passivate them thus making them less accessible to the monomeric portion. Silica sols are easily handled as they are in a liquid form and can be readily fed like the polymeric portion into the reaction vessel without any dust formation. No precipitation of said kind of silica occurs even at the elevated temperatures of the polymerization process.

One can say the more colloidal silica particles are small-sized and homogeneously arranged, the higher the amount of colloidal silica in the copolymers of the invention can be without observing a flaking or precipitating phenomenon in hair treated with said copolymer or a hair styling composition thereof. Higher amounts of colloidal silica will also improve the strength of fixation viz. the strength properties of the inventive copolymers. All this make silica sols to be a silica source of choice for the inventive copolymers. Said copolymers and hair styling compositions thereof particularly enhance the sticking properties of wet hair but are not sticky in dry hair. They provide the capacity of maintaining the hairstyle once fashioned and increase hair fixative properties especially the curl retention.

Silica particles having a small size have the ability to physically and/or chemically connect to an extended amount of monomers of the monomeric portion in particular to monomer B. This is to say that an equal strength of fixation of the copolymer of the invention can be obtained with a smaller amount of colloidal silica having a small medium particle size and a high surface area compared to colloidal silica of greater particle size. This reduces production costs.

A highly preferred colloidal silica of the invention is a silica sol having a weight concentration ranging from 30 to 40 w % colloidal silica, (i.e. 100 g of sol contain 30 to 40 g of colloidal silica), a disodium oxide content ($Na_2O$) ranging from 0.17 to 0.2 g per 100 g of sol and a density of 1.205 to 1.295 $g/cm^3$. It preferably also shows a maximum viscosity of 5 to 20 mPa s, a specific surface area of 200 $m^2/g$ and a mean particle size of 15 nm. Its pH-value ranges from 9 to 9.5 and its storage stability is 18 months. Said silica was found to be highly compatible with the monomers A, B of the invention and yields copolymers which are both highly flexible and tight, viz. have a high strength of fixation.

Another important topic of the invention focuses on the weight ratio between colloidal silica and monomer B in the copolymer of the invention, viz. on the relation between the moiety giving the strength of fixation and the entity having the linker-like properties. Said weight ratio between colloidal silica and monomer B is smaller than or equal to 4:1, preferably smaller than or equal to 2:1 and in a highly preferred embodiment smaller than, equal or slightly higher than 1:1. The fixative properties of the inventive copolymer can only be increased when colloidal silica and monomer B are provided in combination. Best results are obtained with a weight ratio between colloidal silica and monomer B ranging from 0.7:1 to 1.3 to 1. This is as with said ratio the amount of colloidal silica covalently connected to the monomeric portion is the highest thus leading to a very tight and stable copolymer.

Copolymers of the invention owe their high styling performance over an extended period of time to their considerable stability, viz. their tight attachment or connection of monomeric portion and colloidal silica. At least 10 w % of said colloidal silica, preferably 50 w % and more preferably 80 w % thereof is covalently bond to at least one monomer B of the copolymerized monomeric portion. This makes the copolymer fibers formed to get stuck or to jam into each other as already explained and leads to a fibrous structure which would not be obtained without silica particles. Said

fibrous structure can be regarded as to have barbs in form of the colloidal silica particles which mechanically hook into the hairs thus giving part of the styling effect. However as it is only a part of the colloidal silica employed which forms a chemical bond with the monomeric portion, fibers do not get too interwoven, thus to long and can therefore still be easily removed from the hair by means of a mild detergent solution.

In comparative studies silica particles were treated with 3-methacryloyloxy)-propyl trimethoxysilane (MEMO) and said MEMO-shielded particles were thereafter subjected to a radical polymerization with different monomers A. According to this procedure, only 6 w % of the shielded particles were found to be covalently connected to the polymerized monomer(s) A. Said copolymer could not be shown to readily form fibers of the same length and mechanical quality upon tearing on it with a glass rod or a spatula. Not all samples showed a tendency to give fibers, which fibers however were shorter and more labile compared to the copolymers of the invention.

It therefore is an advantageous feature for copolymers of the invention to be synthesized in only one reaction as further detailed below. Copolymers of the invention show a considerable strength of fixation when they are obtained from a one-pot process and at least 10 w % of the colloidal silica used is covalently bond to at least one monomer B of the copolymerized monomeric portion.

Attached silica particles increase the friction between copolymer chains. The higher the silica concentration in the reaction mixture or solvent is, the higher the friction effect between individual copolymer strands formed and the more the copolymer obtained is adapted for having a styling effect viz. a stiffening effect. Said copolymer reaches its maximum stability and styling performance when its solvent is entirely removed. Said copolymers of the invention show a sufficient stability which means they do not break upon considerable bending of the hair.

Different types of colloidal silica were reacted with different monomer B compounds as indicated above. Highly preferred copolymers viz. specimen considerably increasing hair fixative properties are obtained when the colloidal silica is a silica sol and the monomer B is (3-methacryloyloxy)-propyl trimethoxysilane (MEMO) and/or vinyltrimethoxysilane (VTMOS).. Such copolymers enable styled wet hair to maintain its shape even when dried. Also tremendously changing climatic conditions are not prone to disarrange or even to destroy the hairstyle obtained by means of the copolymers of the invention. In particular stiffening effect, stickiness to Kempf, sensory stickiness and curl retention are maintained for quite long times i.e. several days depending on the climatic conditions. Another advantage of said copolymers or hairstyling compositions of the invention is to make the wet hair more sticky yielding the hairstyle to be more stable and being prepared in a faster way.

Another topic of the invention for which independent protection is requested, is a hair styling composition comprising a copolymer having a stickiness according to Kempf at 20 °C and 80 % relative humidity, ranging from 1 to 4, preferably from 2 to 4 and in a mostly preferred embodiment from 3 to 4. Said hairstyling composition preferentially is a hair gel comprising between 75 and 95 w % of deionized water or a hair spray. Stickiness to Kempf of hair styling compositions was determined using a hair gel as disclosed below. Said stickiness reflects the ability of the hairstyling composition freed of excess solvent to still attach to matter brought in contact with it, for instance to particles. This means that said stickiness is not a sensoric one realized upon touching hair with the fingers but purely relates to the composition's capacity to attach to inanimate solid matter. The higher the stickiness values the more efficient the hair styling composition attaches for instance to the hair, and thus the better the styling effect of said composition is.

Hair styling compositions comprising a copolymer of the invention have a viscosity ranging from 24,000 to 45,300 mPA, preferably from 26,900 to 39,100 mPA and in a most preferred embodiment from 27,300 mPA to 29,050 mPA. It was found that hair styling compositions exceeding a value of 45,300 mPA could no longer be easily applied and distributed in wet hair. However compositions with a viscosity of less than 24,000 mPA provided a very thin fluid having the tendency to drain out of the wet hair.

A wider range of colloidal silica and monomer B was shown to be incorporated into the hair styling composition if it is prepared such that its viscosity ranges from 26,900 to 39,100 mPA. However, the highest values for the stiffening effect are obtained with the hair styling composition having a viscosity ranging from 27,300 mPA to 29,050 mPA.

Another aspect of the invention is a fiber comprising a copolymer or a mixture of copolymers of the invention. Said fiber is suitable for the production of receptacles for packaging food or feed products or other goods which require storage under dry conditions. Any humidity reaching the package would disintegrate it prior to attacking the packed good. Receptacles made of said copolymer fibers are biologically highly compatible and do not detoriate nutritional products.

The invention also comprises a process for making the copolymer of the invention. Said process for which independent protection is sought, comprises placing water or an aqueous mixture in a vessel, charging the not yet polymerized monomeric portion or an aliquot II thereof into the vessel and adding at least one initiator to actuate copolymerization. Said monomeric portion of the invention or an aliquot II thereof comprises at least one water-soluble monoolefinic monomer A, at least one monoolefinic silane monomer B, the weight portion of said monomer B being less than 1 % of the total weight of the monomeric portion and contacting said monomeric portion with colloidal silica prior and/or during copolymerization.

The monomeric portion comprises both monomer A and monomer B. Upon contact with colloidal silica both

compounds interact with said colloidal silica thus modifying its particles in a way as to yield the copolymers of the invention. This is to say that colloidal silica, monomer A and monomer B have to be in contact during the polymerization process in order to come to copolymers having the desired strength of fixation and thus to hair styling compositions prepared thereof having the desired stiffening effect. In other words, the advantageous copolymers of the invention and the hair styling compositions thereof are only attained if the colloidal silica particles are added prior or during the polymerization in the presence of at least one vinylic reactive silane monomer.

The addition of colloidal silica as indicated in the last paragraph gives copolymers of the invention which when part of a hair styling composition also considerably preserve the curl retention of a hair style. Said addition of silica prior to or during the polymerization process furthermore leads to copolymers which, when applied as a hair styling composition to the whet hair, make it sticky even during the drying process of the hair. This largely simplifies hair styling. Moreover, upon complete drying of the hair and thus of the hair styling composition, the copolymer stickiness disappears thus giving the hair an agreeable sensoric touch. By using said process for copolymer production, no flaking out of silica particles or complete copolymer components can be observed from the copolymer or the hair styling composition.

However, one obtains copolymers having a considerably reduced strength of fixation as obtained by means of the spatula test and thus hair styling compositions resulting thereof show a largely reduced stiffening effect, if colloidal silica is added to the already entirely copolymerized monomeric portion, as will be shown below. The copolymerization process therefore in no way should be finished prior to contacting colloidal silica with the monomeric portion.

Contacting colloidal silica in a first step with the total amount of monoolefinic silane monomer B and once this reaction finished, with the total amount of monomer A was also found to give different results compared to the process of the invention (cf. supra). The way of conducting such a process and the amounts of colloidal silica as well as the monomers of the polymeric portion used likely have to be adjusted differently for obtaining results comparable to those of the inventive process.

Aliquot I and aliquot 11 within the scope of the invention each mean a certain part of the monomeric portion whereas the monomeric portion comprises monomer A and monomer B as defined supra. Under particular conditions as indicated above, the monomeric portion also comprises monomer C. Aliquot I is placed in the reaction vessel, then treated in a certain manner for instance by heating, and thereafter supplemented with the remaining monomeric portion denoted aliquot II. This is to say that aliquot I and aliquot II together give the monomeric portion. However, the repartition of monomer A and monomer B (and monomer C if required) in aliquot I is not necessarily the same as in aliquot II. Aliquot II can be enriched with monomer B whereas aliquot I contains a reduced amount of said monomer B or vice versa in order to give a concentration for each monomer as required for the monomeric portion in total.

The process for making a copolymer of the invention is realized such that aliquot II comprises between 5 w % and 95 w % of the total weight amount of monomer A present in the monomeric portion and between 5 w % and 95 w % of the total weight amount of monomer B present in the monomeric portion. It is thus possible to get copolymers of the invention having the desired strength of fixation regardless of the respective monomer concentration in each aliquot as long as both aliquots are not entirely depleted of monomer A or of monomer B.

In an extended embodiment of the invention copolymers having an increased strength of fixation translated by an increased stiffening effect in a hair styling composition, could be obtained in a process of the invention wherein aliquot II comprises up to 95 w % of monomer B used in the monomeric portion and up to 5 w % of monomer A used in the monomeric portion. This is to say that even a reduced amount of monomer A in aliquot II and a large amount of monomer B in said aliquot II both already in contact with colloidal silica prior to adding said aliquot II and said silica to the aqueous mixture and realizing the copolymerization process of the invention also give copolymers of the invention having a considerable strength of fixation.

The initiator used within the process of the invention is selected from the group of peroxides and/or the group of azo initiators.

The peroxides comprising both organic and inorganic peroxides are dibenzoyl peroxide, diacetyl peroxide, succinyl peroxide, tert-butyl perpivalate, tert-butyl 2-ethylhexanoate, tert-butyl permaleate, bis-(tert-butylperoxy)cyclohexane, tert-butylperoxy isopropyl carbonate, tert-butyl peracetate, 2,2-bis(tert-butylperoxy)butane, dicumyl peroxide, di-tert-amyl peroxide, di-tert-butyl peroxide, p-menthane hydroperoxide, pinane hydroperoxide, cumene hydroperoxide, tert-butyl hydroperoxide, hydrogen peroxide sodium persulfate and mixtures of said initiators. Said initiators can also be used in combination with redox components such as ascorbic acid. Particularly suitable peroxide initiators are tert-butyl perneodecanoate, tert-butyl perpivalate or tert-butyl 2-ethylhexanoate.

Preferably, the free-radical initiators are azo initiators. Suitable azo initiators are ,2,2'-Azobis[2-(2-imidazolin-2-yl)propane]dihydrochloride, 2,2'-Azobis[2-(2-imidazolin-2-yl)propane]disulfate dihydrate, 2,2'-Azobis(2-methylpropionamidine)dihydrochloride, 2,2'-Azobis[N-(2-carboxyethyl)-2-methylpropionamidine]hydrate, 2,2'-Azobis{2-[1-(2-hydroxyethyl)-2-imidazolin-2-yl]propane}dihydrochloride, 2,2'-Azobis[2-(2-imidazolin-2-yl)propane], 2,2'-Azobis(1-imino-1-pyrrolidino-2-ethylpropane)dihydrochloride, 2,2'-Azobis{2-methyl-N-[1,1-bis(hydroxymethyl)-2-hydroxyethl]propionamide, 2,2'-Azobis[2-methyl-N-(2-hydroxyethyl)propionamide].

In a preferred embodiment of the invention the initiator added is a pair of initiators comprising a peroxide initiator

and an azo initiator. This gives the opportunity to conduct the inventive process at various temperatures as peroxides act only at high temperatures as initiators whereas azo initiators already do at lower ones.

In a highly preferred embodiment the initiator added comprises a peroxide initiator, an azo initiator and sodium disulfite as said sulfite confers the peroxide to already act as initiator at temperatures even lower than those required for the azo compound. Said activity at lower temperatures is due to the ability of said disulfite to reduce the peroxides and thus to generate radicals as intermediates. Said sodium disulfite is also used at the end of the inventive process to reduce any radicals still existing.

Water within the scope of the inventive process comprises ordinary tap water as well as destilled or deionized water. In a preferred embodiment of the invention the water used is deionized water obtained by passing tap water over an ion-exchange resin unit. This is convenient as ion-depleted water will not have any influence on the process of the invention.

An aqueous mixture as required for the inventive process is a mixture of different solvents containing inter alia water. In a preferred embodiment the aqueous mixture is a mixture selected from the group consisting of water and soluble or water-dispersible solvents comprising methanol, ethanol, n-propanol, isopropanol, ethylene glycols, propylene glycols, ethandiol and glycerol.

In a special embodiment of the invention the aqueous mixture contains traces of water. They are for instance present in methanol or ethanol. This is necessary when a large aliquot I of the monomeric portion is placed in the aqueous mixture in order to avoid increased hydrolysis of monomer B to take place prior to the copolymerization process. The same applies for an aliquot I containing a large amount of monomers belonging to the group of monomer B.

For certain copolymers of the invention the copolymerization process cannot be realized with the entire monomeric portion in the presence of colloidal silica (batch process) as either said portion and colloidal silica get not readily dissolved or the polymerization process will not start or it will be inhomogeneous despite thorough stirring or finally it will be too fast to be controlled. To overcome this, water or the aqueous mixture contains at least one water-soluble monoolefinic monomer A. This means monomer A or one of the monomers comprised within monomer A are placed in the reaction vessel prior to adding aliquot II of the monomeric portion. This is a semi batch or feeding process wherein aliquot II is suitable to be metered continuously (feeding) or in distinct steps (semi batch).

In a more specified embodiment the at least one water-soluble monoolefinic monomer A in water or in the aqueous mixture comprises N-vinylpyrrolidone the amount of which ranges from 12 to 18 w % and preferably from 14 to 16 w % of the total amount of N-vinylpyrrolidone used. As N-vinylpyrrolidone makes the major part of monomers within monomer A, it is crucial to get it well distributed in the copolymer of the invention in order to get a homogenous copolymer of the invention in a cost-effective way. For that reason the indicated amount is separated from the monomeric portion and placed in the aqueous mixture.

In another embodiment of the invention not only monomer A or some of the monomer-members selected out of monomer A are placed in the vessel. Instead water or the aqueous mixture containing an aliquot I of the monomeric portion, said aliquot I making 4 to 25 w % of the total weight amount of the monomeric portion, preferably 6 to 18 w %, more preferably 8 to 14 w % and in a particularly preferred embodiment 10 to 12 w % are used for this. Such kind of measure is necessary if one works in concentrated solutions in order to overcome the drawbacks of solubilization, of slow polymerization start or of inhomogeneous or to fast polymerization which would increase processing costs. It was found that placing between 8 and 14 w % of the monomeric portion in the aqueous mixture both increases the copolymerization rate and reduces the energy required for stirring means. Placing the aliquot I in the aqueous mixture also servers for initiating the polymerization process with a small amount which process then is to progress continuously. This gives very homogeneous copolymers of the invention in a cost-effective manner.

Yet an extended version of the inventive embodiment mentioned in the last paragraph provides the water or the aqueous mixture to contain an aliquot S, said aliquot S making between 2 and 8 w %, preferably 5 w % of the total weight amount of colloidal silica sol used. This measure is important to come to highly homogeneously formed and intermingled copolymers of the invention.

Still a more developed embodiment of the invention comprises in addition to the features mentioned in the last two paragraphs, the water or the aqueous mixture to contain an aliquot P of a proton donor, said aliquot P ranging from 2 to 8 w %, preferably being 5 w % of the total weight amount of the proton donor used. Said proton donor preferentially is phosphoric acid. In doing so, condensation of aliquot S of the colloidal silica and of aliquot I of monomer B of the monomeric portion takes place prior to or in the beginning of the copolymerization process of aliquot I of the monomeric portion leading to raw copolymers of the invention considerably crosslinked to colloidal silica. Said copolymers provide an extensive setting performance in hair styling compositions applied to wet hair.

An even more elaborated embodiment of the invention comprises in addition to the features of the last three paragraphs, the water or the aqueous mixture to contain an aliquot A said aliquot A being an aliquot of an azo initiator and said aliquot A making between 2 and 10 w % and more preferably between 5 and 6 w % of the total weight amount of the azo initiator used. By said means both condensation of monomer B with colloidal silica and copolymerization of the monomeric portion takes place simultaneously already in water or in the aqueous mixture placed in the vessel and

helps to smoothly initiate the process for making a copolymer of the invention.

Another feature to quickly come to homogeneous copolymers of the invention and thus to make the inventive process more straight forward and more cost-effective is to use at least one water-soluble monoolefinic monomer A in diluted form. Said monomer A is methacrylamide. Said methacrylamide is used as aqueous solution having a concentration ranging from 10 to 20 w % and preferably being 15 w %.

However monomer A and monomer B (and also monomer C if present) are not required to be allotted in the same amounts to aliquot I as in the monomeric portion. For instance monomer B or representative molecules thereof are apt to be present in aliquot I in higher concentrations than in the monomeric portion in total.

In another embodiment of the invention the not yet polymerized monomeric portion or aliquot II thereof comprises a proton donor. In a preferred embodiment said proton donor is phosphoric acid. The presence of said up to three protons liberating acid leads monomer B to more readily hydrolyze to an analogue of silicic acid and said silicic acid in part chemically connects to colloidal silica said condensation process being driven by the formation of water. This leads to copolymers of the invention providing a high strength of fixation as explained supra. Proton donors liberating less protons are less preferred.

It is to be understood that said proton donor and said phosphoric acid are suitable to be used within each embodiment of the inventive process even if it is not expressively disclosed within each such embodiment. Each embodiment of the inventive process is to be red either with proton donor preferably with phosphoric acid or without proton donor.

In a standard process of the invention the proton donor and at least one initiator are reacted simultaneously with colloidal silica, the not yet polymerized monomeric portion or an aliquot II thereof and water or the aqueous mixture. In doing so, copolymerization of monomer A or of monomer A and C with monomer B and hydrolysis of the silylorganic moiety of monomer B followed by partial condensation to colloidal silica takes place simultaneously.

However, in a modified embodiment of the inventive process, water or the aqueous mixture the not yet polymerized monomeric portion or an aliquot II thereof and the colloidal silica are reacted with the proton donor and incubated for a period of time prior to adding at least one initiator. Said period of time ranges from 5 min to 120 min. This measure is taken in order to let the hydrolysis process of monomer B proceed further prior to starting copolymerization yielding the rate of colloidal silica to be covalently bond to monomer B to be higher. Thus by adjusting the period of time for contacting colloidal silica, the monomeric portion, the aqueous mixture and phosphoric acid with at least one initiator will influence on the structure and thus on the strength of fixation of the copolymers of the invention. One should take care to add the at least one initiator prior to term of the hydrolysis process of monomer B as mentioned supra.

In a highly preferred embodiment of the inventive process the weight amount of colloidal silica used ranges from four times to one time the weight amount of monoolefinic silane monomer B, preferably from two times to one time the weight amount of monoolefinic silane monomer B and in a mostly preferred embodiment being equal to the weight amount of monoolefinic silane monomer B employed. These ratios of silica and monomer B gave copolymers of the invention having a high strength of fixation.

Fast copolymerization of the monomeric portion in the presence of silica was observed when the water or the aqueous mixture is heated up to 60 to 80 °C preferably up to 70 °C under an inert atmosphere. Working under said inert atmosphere reduces and mostly completely avoids the formation of oxidation side-products prior or during the copolymerization process. An inert atmosphere comprises gases selected from the group comprising nitrogen, argon, xenon, helium or mixtures thereof. The inert atmosphere also comprises air/nitrogen mixtures having a nitrogen amount of at least 50 w %. However, such air containing mixtures are preferentially used with diluted monomeric portions and are less adapted to concentrated ones.

Most homogeneous copolymers of the invention were obtained when the monomeric portion or the aliquot II thereof is charged into the water or into the aqueous mixture in a period of time ranging from 3 h to 5 h, preferably 4 h. This feed process is highly recommended with the amounts of silica and monomeric portion used (cf. examples below) as it assures a smooth and regular reacting of the monomers of monomer A (of monomer C if provided), of the monomers of monomer B and of colloidal silica.

One particular aspect of the inventive process is to use the initiator in an amount of 0.01 to 10 w % and preferably of 0.3 to 5 w % of the amount of the monomeric portion. This both makes the copolymerization reaction sufficiently fast and on the other hand will not give to many copolymers having a short chain thus reducing the strength of fixation of the copolymer which will take place upon increasing the amount of initiator or initiators.

The initiator is added at a vessel temperature ranging from 70 °C to 80 °C. Working at said temperatures immediately activates the azo initiator to form radicals and slowly shifts the peroxides to do so thus giving an almost homogeneous repartition of radical initiators along the copolymerization process. This gives highly homogenous copolymers of the invention having said extraordinary strength of fixation.

It is also crucial for the process of the invention to add the at least one initiator into the vessel over a period of time which is between 1 and 1.3 times the period for charging the not yet polymerized monomeric portion. If it takes for instance 4 h to add said monomeric portion into the vessel, the at least one initiator is added during 4 h 30 min. By this

measure, one can achieve an almost entire copolymerization making the further processing of the copolymer obtained easier and thus more cost-effective.

Conducting of the process of the invention is largely simplified and cost-saving, if the colloidal silica used is a silica sol. This is as a silica sol can be readily added like a solvent into the vessel without any formation of hazardous dust or of silica aggregation as may happen for other forms of silica.

An essential feature of the inventive process is to supply the monomeric portion and colloidal silica from a first inlet and to add the free radical initiator to the reaction vessel from at least one inlet different from said first inlet. This gives the opportunity to also physically separate the copolymerization reaction of monomer A (or of monomer A and C) and monomer B and the hydrolysis and condensation reaction of monomer B and colloidal silica if need be. Having said option puts the practitioner in a position to always obtain the same quality of copolymers of the invention regardless of the copolymer amount required or the nature of the monomeric portion.

For standard purposes the process of the invention requires the monomeric portion, colloidal silica and at least one initiator to be mixed together by feeding preferably under acidic conditions. Said feeding means starting to add colloidal silica, the monomeric portion and at least one initiator into the vessel is realized simultaneously. This way both the copolymerization process as well as the condensation process of colloidal silica to monomer B takes place simultaneously even if the monomeric portion, colloidal silica and at least one initiator are supplied from different inlets. Such protocol gives time savings and thus leads to a lean and therefore cost-effective process of the invention

Adding the free radical initiator to actuate polymerization from at least one inlet different from the first inlet advantageously within a time which is 1 to 1.25 preferably 1 to 1.125 fold the time required for supplying the monomeric portion and colloidal silica from the first inlet. This is to say that the free radical initiator is continuously fed into the vessel along with the monomeric portion and colloidal silica even after the supply of monomeric portion and colloidal silica being terminated. By said measure, viz. the addition of initiator in excess, one assures complete copolymerization of the monomeric portion in the presence of colloidal silica. No traces of unreacted monomer A, monomer B and monomer C if present will remain and hamper the copolymers of the invention to be used in the field of body care especially for hair styling purposes. Thus no further laborious and expensive removal of monomers by e.g. distillation, stripping etc. from the crude copolymer obtained is required.

However, if large quantities of monomer/monomers A and monomer/monomers B and of colloidal silica are to be reacted, or copolymers of the invention are required having an extremely small range of molecular weights, or if a narrow concentration range of colloidal silica is required to be covalently bond to the monomeric portion, a different feeding protocol is required for cost-effective copolymerization. The invention than provides a process wherein the monomeric portion and colloidal silica are supplied from a first inlet and the free radical initiator is added to the reaction vessel from at least one inlet different from the first inlet at a moment when from10 w % to 100 w % of the monomeric portion and of the colloidal silica are already charged into the vessel. Yet another advantageous embodiment of the inventive process describes the monomeric portion and colloidal silica to be supplied from a first inlet and the free radical initiator being added to the reaction vessel from at least one inlet different from the first inlet 5 to 10 minutes after the monomeric portion and the colloidal silica were entirely charged into the vessel. One chooses this latter way of retarded addition of the free radical initiator to the reaction mixture containing the monomeric portion and colloidal silica in order to get a large part of monomer B and colloidal silica reacted prior to initiating the copolymerization process of monomer A and monomer B and monomer C if present. This method is also used in case of poorly reactive types of monomer B not readily interacting with colloidal silica. It is hardly possible to obtain copolymers of the invention from poorly reactive monoolefinic silane monomers B in a likely cost-effective way without postponed addition of the radical initiator.

Realizing the process of the invention at room temperature would be rather time consuming and thus expensive. The copolymerization is thus carried out at temperatures ranging from 50 °C to 220 °C, preferably from 50 °C to 100 °C. Working only up to 100 °C gives the opportunity to use not specially adapted and thus inexpensive recipients.

Another embodiment of the inventive process requires the copolymerization to be realized under pressure at temperatures from 95 °C up to 220 °C. Said feature of applying pressure is required in case of working with monomeric portions solubilized in small quantities of solvent or not solubilized at all. With such small solvent amounts at higher temperatures as claimed, the aqueous mixture, the liquid phase of the colloidal silica and the solvent of the monomeric portion will tend to evaporate which only can be avoided by means of applying pressure.

Another essential feature of the inventive process is to synthesize the copolymer by radical-polymerization in solution. Copolymers having such a remarkable strength of fixation were obtained from solution systems. Equal results could not be obtained by means of copolymerization in dispersions. This is probably due to a lesser degree of mixing of the monomeric portion and colloidal silica and the at least one initiator, thus giving a less extensive contact between said components.

The invention further comprises the use of a copolymer, comprising colloidal silica and a copolymerized monomeric portion of at least one water-soluble monoolefinic monomer A, and at least one monoolefinic silane monomer B for cosmetic purposes particularly for hair styling purposes. Such copolymers by now were not likely to be used as hairstyling means.

Further features, details and advantages of the invention arise from the wording of the claims and from the following description disclosing embodiments and examples of the invention. Said description also discloses a general way of obtaining the copolymers of the invention followed by detailed process examples. A further topic deals with the performance of said copolymers in hairstyling compositions and the preparation thereof.

Monomer A or the monomers to be understood under monomer A, monomer B or the monomers understood under monomer B, eventually also monomer C or the monomers understood therewith and colloidal silica are introduced together with the initiator into a stirred reactor or vessel at the polymerization temperature. The initiator prior to introduction into the vessel or the reactor is solubilized in an appropriate solvent. Introduction of monomer A and monomer B, viz. the monomeric portion, of colloidal silica, of an appropriate solvent containing a proton donor and of the solubilized initiator is realized by means of a feeding process. This is to say that the different components are administered over a period of time which is different for water or the aqueous mixture, for the not yet polymerized monomeric portion and for the at least one initiator.

Aliquot I of the monomeric portion, water, an aliquot S of the colloidal silica, an aliquot P of a proton donor and an aliquot A of an azo initiator are placed in the vessel which is a reactor adapted to support internal pressures up to several bars. Said preparation of aliquots is thereafter defined as raw mixture. Said raw mixture can also be understood as aqueous mixture supplemented with aliquot S, aliquot P and aliquot A.

Aliquot I of the monomeric portion comprises methacrylamide, 1-vinylimidazole, N-vinylpyrrolidone and methacryloyloxy)-propyl trimethoxysilane (MEMO). It makes from 10 to 12 w % of the monomeric portion. However the weight distribution of said monomer(s) A and said monomer B is not identical to the overall weight distribution of said monomers in the monomeric portion in total. The percentage amount of aliquot I from the monomeric portion is calculated as follows from the data given in the examples below: The total weight amount of all components present in the process of the invention with the exception of water the radical initiators and sodium disulfite is determined. Then the total weight amount of the monomeric portion is determined and deduced from the total weight amount. The remainder makes a certain percentage of the total weight amount. Then the value making 5 w % of the total weight amount of water-depleted feed 1 (definition of feed 1 cf. below) is calculated. Said value is multiplied with the certain percentage mentioned above and gives the portion within said value not belonging to the monomeric portion. Deducing said portion from the value making 5 w % of the total weight amount of feed I gives the amount of aliquot I of the monomeric portion in feed I. Besides said aliquot I in feed I one or more further monomers are in the raw mixture. The sum of these monomers and of aliquot I in feed I gives the aliquot I of the monomeric portion present in the raw mixture. Putting the amount of aliquot I in relation to the weight amount of the monomeric portion gives the percent amount of aliquot I to range between 10 and 12 w % of the monomeric portion. The way of calculation will be exemplified below with one of the embodiments of the invention.

Aliquot S is an aliquot of the weight amount of the colloidal silica sol used. Said aliquot makes between 2 and 8 w %, preferably 5 w % of the total weight amount of colloidal silica sol used and its calculation will be shown below. The colloidal silica used is a silica sol with the silica having a particle size of 15 nm and a specific surface area of 200 $m^2/g$. It has a density of 1.295 $g/cm^3$ and a maximal viscosity of 20 mPa s. Its concentration in water is 40 w% and its $Na_2O$ content is 0.2 w %.

Aliquot P denotes the amount of proton donor to be placed in the reaction vessel. Said aliquot P ranges between 2 and 8 w %, preferably 5 w % of the total weight amount of proton donor used and its amount will be calculated below by way of example. A preferred proton donor used is phosphoric acid in a concentration of 85 w %.

Aliquot A is an aliquot of an azo initiator which in several cases is 2,2'-azobis(2-methylpropionamidine)dihydrochloride also known as Wako V 50. Said aliquot makes between 5 and 6 w % of the total weight of the azo initiator as will be exemplified below.

The raw mixture as disclosed supra is heated up to 70 °C under an inert atmosphere which preferably is nitrogen. This leads the components of the raw mixture to partially copolymerize and arranges for the large amount of one component of monomer A used, viz. N-vinylpyrrolidone to be homogeneously distributed within the forming copolymer of the invention. The amount of N-vinylpyrrolidone in the raw mixture ranges from 14 to 16 w % of the total amount of N-vinylpyrrolidone used in the monomeric portion the calculation of said values will be exemplified below.

Said heated raw mixture is then simultaneously supplemented with the remaining portion of feed 1 (not yet part of aliquot I, aliquot S and aliquot P) and with the remaining portion of feed 2 (not yet part of aliquot A).

Feed 1 comprises colloidal silica, the proton donor, deonized water and aliquot II of the monomeric portion, with the monomeric portion consisting of methacrylamide, 1-vinylimidazole, N-vinylpyrrolidone, and methacryloyloxy)-propyl trimethoxysilane (MEMO). For all embodiments of the invention the weight portion of the monomer N-vinylpyrrolidone of monomer(s) A in aliquot II is less than in the monomeric portion in total. Feed 1 is administered into the vessel during 4 h for all embodiments of the invention.

Feed 2 comprises azo initiator 2,2'-azobis(2-methylpropionamidine)dihydrochloride in deionized water. The concentration of azo initiator in deionized water both in feed 2 and in aliquot A is identical and ranges from 2 to 4 w %. Feed 2 is administered into the vessel during a time of 4 h 30 min for all embodiments of the invention. This is to say that administering of the azo initiator of feed 2 is continued for another 30 minutes after termination of feed 1. By said

way of proceeding, the amount of monomers remaining in the vessel can be considerably reduced thus making the further work-up of the copolymers of the invention much easier, said work-up being necessary in order to comply with requirements for body care products, especially hair cosmetics.

The reaction mixture obtained is stirred for another 2 h at 70 °C for completion of the reaction and than heated at 80 °C. At said temperature the reaction mixture is supplemented with feed 3 in one portion and stirred another 10 min. Feed 3 is an aqueous solution of the radical initiator tert-butylhydroperoxide the concentration of which ranges from 2 to 3 w %. This initiator converts any not yet polymerized monomers.

Eliminating of radical initiators and still persisting copolymer radicals is then realized by means of feed 4. Said feed 4 is either continuously introduced into the vessel during 30 min or in 4 portions every 5 min one. Feed 4 is an aqueous solution of sodium disulfite. The concentration of sodium disulfite in feed 4 ranges from 10 w % to 12 w %. After another 2 h of stirring the reaction mixture is heated up to 120 °C, subjected to a water vapor distillation for about 1 h and cooled to 40 °C.

The copolymers of the invention thus obtained have the aspect of a highly viscous slightly turbid solution.

They are monitored to have a sufficient strength of fixation by the already explained spatula test. However, in addition thereto hairstyling compositions are prepared and said hair styling compositions are subjected to various physical and sensoric tests in order to demonstrate the capacity of the inventive copolymers therein to improve the stickiness, the sensory stickiness, the stiffening effect, the bending retention and the curl retention of hair treated with said hair styling composition. One also determines physicochemical parameters as the viscosity of the hair styling composition and the K-value of the inventive copolymer.

Determination of the K-value of copolymers of the invention:

K-values were determined as disclosed in Fikentscher, Cellulosechemie, Vol. 13, p. 58 to 64 (1932) at 25 °C in a solution of N-methyopyrrolidone (NMP-solution). Said K-values provide a means to determine the molecular weight of the copolymer of the invention. An exact amount of 1.000 g of dry solid of the copolymer of the invention is placed in a volumetric flask of 100 ml and dissolved by adding the NMP-solution and shaking. The solution obtained is supplemented with further NMP-solution up to the calibration mark of the volumetric flask. Said solution is placed in a capillary viscosimeter according to Ubbelohde and the latter being incubated for 30 min at 25 °C after mounting it into a thermostat. The flowtime of the solution between the two calibration marks of the viscosimeter is then determined several times and averaged. The value obtained is then corrected by the Hagenbach-Couette coefficient provided with the viscosimeter. The same measurement is repeated with the pure NMP-solution and the ratio $\eta_{rel}$ is determined by dividing the flowtime value of the copolymer supplemented NMP-solution by the flowtime value of the pure NMP-solution. From said ratio $\eta_{rel}$ the K-value is determined by means of a conversion table as disclosed for instance in the handbook of Wilborn, Physikalische und Technische Prüfverfahen für Lacke und ihre Rohstoffe, Edition of 1953.

Preparation of a gel, viz. one hair styling composition of the copolymers of the invention:

An aqueous solution containing 1 % of Carbopol® 940 (CAS No. 9003-01-4 acrylic acid polymer) was prepared as follows: 9880 g of deionized water and 20 g of Euxyl® K 100 (antibacterial preparation comprising benzyl alcohol, methylchloroisothiazolinone and methylisothiazolinone and having a refractive index $n_d^{20}$ of approximately 1.428 and a density at 20 °C of 1.07 g/cm$^3$) were placed in a beco-homogenizer. The homogenizer was started and adjusted such that a funnel forms in the preparation. 100 g of Carbopol® 940 were quickly added to the preparation which was further homogenized for at most 10 min. The homogenizer was than tightly closed and the stirrer started. Stirring was to be realized until all clots have disappeared.

An aqueous (if required neutralized) solution containing 6 w % of dried copolymer of the invention was prepared now referred to as copolymer solution.

98.66 g of the Carbopol® 940 solution previously prepared were neutralized with triethanolamine and stirred for approximately 30 min at 80 to 100 RPM with a paddle mixer to form a gel. The copolymer solution was than added to said Carbopol® 940 solution by means of a dropping funnel for a period of 30 min with constant stirring and further stirring for at least another 30 min. The gel thus formed was centrifuged and evaluated after 24 h.

Determination of the viscosity of hair styling compositions of the invention:

The viscosity of the hairstyling composition of the invention was measured at room temperature in mPa s by means of a Brookfield RVT viscosimeter into which the gel as indicated supra was injected.

Determination of the stiffening effect of a hair styling composition treated- viz. a gel-treated hair strand:

Preparation of a gel for hair styling purposes comprising the copolymer of the invention was realized as indicated under "Preparation of a Gel...".

1 g of said gel was applied onto a hair strand having a length of approximately 23 cm by means of a spatula and spread in the hair strand's direction with the fingers. Said strand dried over night at room temperature and was analyzed by at least two persons the next day. The results obtained are ranged as follows:

| | |
|---|---|
| +++ | very good stiffening effect |
| ++ | good stiffening effect |
| + | still good stiffening effect |
| O | poor stiffening effect |

Stickiness according to Kempf of a hair styling composition of the invention:

The gel to be analyzed, viz. the gel comprising a copolymer of the invention and prepared as indicated supra was applied as a thin layer onto a glass plate. Once the layer dried to form a film, the film supplemented glass plate was stored over night at 20 °C and 80 % of relative humidity in a climatic cabinet. Said plate was then placed onto a Kempf apparatus. 5 mm above the film obtained from the gel a tape was mounted to the apparatus, said tape being a plastic-carbon tape of Pelikan 2060 type having a width of 50 mm. By means of a round rubber stamp (diameter 400 mm, hardness according to Shore A of 60 ± 5) said tape was pressed onto the film for 10 sec. with a force of 250 ± 5 N. The more the film surface is sticky the more particles of the plastic-carbon tape will remain on the film.

The evaluation ranges from 0 to 5 such that the respective values are to be understood as follows:

| | |
|---|---|
| 5 | extremely sticky |
| 4 | very sticky |
| 3 | sticky |
| 2 | less sticky |
| 1 | hardly sticky |
| 0 | not sticky at all |

Measurement of the sensory stickiness of a hair strand treated with a hair styling composition of the invention:

Said stickiness was determined with moistened hands. 1 g of the gel of the invention as shown supra in "Preparation of a gel..." was applied onto each side of a hair strand and combed into said strand which has a width of 6 cm and a length of 23 cm (thus 2 g of gel in total were applied). The strand was dried for at least 3 h after which the stickiness thereof was determined by at least two persons. They moistened their hands by means of a wet window cloth for 10 sec. after which they compressed the hair strand in the hand for 10 sec.. Upon releasing said strand from the hands the sensory stickiness was evaluated. For means of comparison at least one standardized gel was used.

Evaluation is made in a range from 0 to 3 as follows:

| | |
|---|---|
| 0 | not at all sticky |
| 1 | hardly sticky |
| 2 | sticky |
| 3 | extremely sticky |

Determination of the flexural strength Bt of a hair strand treated with a hair styling composition of the invention:

A gel of the copolymer of the invention as indicated supra was prepared as hair styling composition. 50 g of said gel were dissolved in deionized water to get a solution of 220 ml. A dried hair strand having a length of 24 cm was weighed. 3 g thereof were immersed in the solution previously prepared, removed thereof and excess solution was stripped. Immersing, removing and stripping was realized three times in order to achieve a homogeneous reparation of the solution in the hair strand.

The last stripping of excess solution was done with thumb and forefinger. Further removal of the solution was realized by pressing the hair strand between filter paper such that the hair strand's weight increases by 1 to 1.4 g (with regard to the initial weight of the hair stand). The strand thus obtained was arranged in order to have a round cross-section and was stored at 20 °C and 65 % of relative humidity over night in a climatic chamber.

Analysis of the strand prepared was realized in the climatic chamber having the climatic conditions mentioned before by means of a pull and compression analyzer (Easytest 86 8002, Fa. Frank). The hair strand was placed in a symmetrical way on two cylindrical rolls of the sample holder, said rolls having a diameter of 4 mm and being separated from each other by a distance of 9 cm). By means of a rounded stamp approached from the upper side of the strand and in the exact middle thereof the strand was bent by 40 mm which leads to fracture the gel film obtained on the strand. The force required therefore is determined in N by means of a load cell.

Determination of the curl retention C.R. of a strand treated with a hair styling composition of the invention:

A gel as indicated above containing a copolymer of the invention was prepared and a sufficient amount of said

hair styling composition was applied onto a glass plate. By means of a spatula the gel was also spread over a hair strand in a way to uniformly saturate said strand previously washed and dried with the gel. Excess gel was squeezed out and combed out. The strand having a length L of 15.5 cm was then wound on a Teflon® rod having a diameter of 12 mm and fixed thereto by means of filter paper and elastics. The fixed strand was then dried over night at a temperature ranging from 60 °C to 70 °C.

The strand supplemented rod was cooled down for 30 min. and at room temperature the curl obtained was carefully removed therefrom in order to avoid the gel film formed thereon to break. The curl was suspended and the curl length L0 measured. Thereafter the curl was placed in a climatic chamber having a temperature of 25 °C and a relative humidity of 90 %. The curl length Lt was determined after 15, 30, 60 and 90 min. as well as after 2, 3, 4, 5 and 24 h of incubation in said climatic chamber. Length measures were realized for 5 strands respectively and mean values Lt obtained after 5 h were used to determine the curl retention using the following equation:

$$\text{Curl-retention C.R. in \%} = (L - L_t) / (L - L_0) \times 100$$

In order to show the different properties of copolymers of the invention and the behavior of their respective gels, several copolymers were prepared as shown in table 1 and introduced in a hair styling composition.

Table 1

| Example | N-Vinyl-2-pyrrolidone | | Methacrylamide | | 1-Vinyl-imidazol | | MEMO (1) | | Colloidal Silica (2) | | K-Value | Viscositiy [mPA] | Stiffening Effect | Stickiness Kempf 20 °C, 80 % r F | Sensory Stickiness | Bt [cN] | CR [%] |
|---|---|---|---|---|---|---|---|---|---|---|---|---|---|---|---|---|---|
| | [g] | [w%] | [g] | [w%] | [g] | [w%] | [g] | [w%] | [g] | (4) | | | | | | | |
| 1 | 183 | [73 2] | 50 | [20] | 15 | [6] | 2 | [0 8] | 2 | [0.8] | 75 7 | 27,300 | +++ | 3-4 | 2 | 206 | 85 |
| | | | | | | | | | | | | | | | | | |
| 2 | 184 | [73 6] | 50 | [20] | 15 | [6] | 1 | [0 4] | 2 | [0 8] | 70 6 | 29,050 | ++ | 1-2 | 4 | 160 | 75 |
| | | | | | | | | | | | | | | | | | |
| 3 | 184 5 | [73.8] | 50 | [20] | 15 | [6] | 0.5 | [0 2] | 2 | [0.8] | 65 3 | 26,900 | + | 2-3 | 2 | 199 | 86 |
| | | | | | | | | | | | | | | | | | |
| 4 | 183 | [73 2] | 50 | [20] | 15 | [6] | 2 | [0 8] | --- | | 71 5 | 30,450 | + | 2 | 2 | 283 | 95 |
| 4a. | 183 | [73 2] | 50 | [20] | 15 | [6] | 2 | [0 8] | 2 (5) | [0 8] | | 38,100 | + | 2 | 2 | 233 | 94 |
| 5 | 183 | [73 8] | 50 | [20 2] | 15 | [6] | --- | | --- | | 68 7 | 32,700 | + | 2 | 2 | 306 | 81 |
| 5a. | 183 | [73 8] | 50 | [20.2] | 15 | [6] | --- | | 2 (5) | [08] | | 36,050 | + | 2 | 2 | 162 | 85 |
| 6 | 183 | [73.2] | 50 | [20] | 15 | [6] | --- | | 2 | [0.8] | | | | | | | |
| 6a | 185 | [74] | 50 | [20] | 15 | [6] | --- | | 2 | [0 8] | 68 4 | 39,100 | + | 2-3 | 2 | 232 | 91 |
| 6b | 185 | [74] | 50 | [20] | 15 | [6] | --- | | --- | | 77.0 | 45,300 | + | 3 | 2-3 | 219 | 95 |
| 7 | 160 + 25 (3) | [74] | 50 | [20] | 15 | [6] | --- | | --- | | 82 0 | 48,900 | + | 2 | 1-2 | 280 | 96 |

[ ] = numbers in brackets are respective weight percentage values of the monomeric portion, and the monomeric portion is given for each example by the sum of weight values indicated in columns 2 to 5
(1) = 3-methacryloyloxy)-propyl trimethoxysilane
(2) = Levasil 200 A
(3) = 160 g N-vinyl-2-pyrrolidone and 25 g N-vinyl-ε-caprolactam
(4) = Amount of colloidal silica added per 100 g of monomeric portion
(5) = colloidal silica was added into the polymer solution after polymerization

One realizes from table 1 an overall effect given by the stiffening effect, the sensory stickiness and the stickiness according to Kempf (viz. in the moist state) to be very high for hair styling compositions of examples 1 to 3, comprising copolymers obtained from the monomeric portion which is polymerized in the presence of colloidal silica. This is to say that monomer A and monomer B (and monomer C if present) have to be contacted with colloidal silica prior or during polymerization. Said overall effect strongly increases the hair fixative properties of the hair styling composition viz. provides a copolymer which shows a strong setting performance.

The overall effect is considerably reduced without using colloidal silica or by adding it once polymerization is finished (cf. examples 4, 4a). This effect is even worse if the monomeric portion also lacks monomer B (cf. examples 5, 5a).

A reduced overall effect of the hair styling composition is also obtained with copolymers only lacking monomer B (cf. examples 6a and 6b) and said drawback cannot be abolished by means of merely changing the monomer composition of monomer A as shown in example 7.

It is further to be noted that the ratio of monomer B viz. MEMO and colloidal silica influences on the strength of fixation of the copolymer formed and thus on the stiffening effect of a hair styling composition of said copolymer. Said weight ratio between colloidal silica and monomer B is smaller than or equal to 4:1, preferably smaller than or equal to 2:1 and in a highly preferred embodiment smaller than, equal or slightly higher than 1:1.

Table 1 also shows monomer B to be less than 1 w% of the total weight of the monomeric portion.

Per 100 parts by weight of monomeric portion the copolymer of the invention comprises one part by weight or less and preferably at least 0.8 parts by weight of colloidal silica.

Example 1, 2 and 3 show copolymers of the invention comprising N-vinyl-2-pyrrolidone also named N-vinylpyrrolidone, methacrylamide, 1-Vinyi-imidazol, 3-methacryloyloxy)-propyl trimethoxysilane (MEMO) and colloidal silica.

In example 1 a raw mixture comprising

| | |
|---|---|
| Deionized water: | 362.00 g |
| N-vinylpyrrolidone: | 17.5 g |
| 5 w % of feed 1: | 25.95 g |
| 5 w % of feed 2: | 2.73 g |

is placed in the vessel which is a reactor.

Feed 1 comprises:

| | | |
|---|---|---|
| Methacrylamide (MAM) | 333.33 g | aqueous solution containing 15 w % of MAM |
| Phosphoric acid | 1.25 g | aqueous solution containing 85 w % of phosphoric acid |
| 1-vinylimidazol | 15 g | |
| N-vinylpyrrolidone | | 165.5 g |
| 3-methacryloyloxy)-propyl trimethoxysilane (MEMO) | | 2 g |
| colloidal silica | 2 g | aqueous solution containing 40 w % of colloidal silica particles, |

the colloidal silica being a silica sol having a weight concentration ranging from 30 to 40 w % of solid silica and a specific surface area of 200 $m^2$/g,

Feed 2 comprises:

| | |
|---|---|
| Deonized water | 52.5 g |
| Wako V 50 | 2 g |

Said raw mixture comprises an aliquot I of the monomeric portion, water, an aliquot S of the colloidal silica, an aliquot P of a proton donor and an aliquot A of an azo initiator. It can also be understood as aqueous mixture supplemented

with aliquot P, aliquot S and aliquot A.

The amount of aliquot I of the monomeric portion is calculated as explained above and quantified infra:

(1) Total weight amount of all components as defined supra:

$$17.5 \text{ g} + (333.33 * 0.15) \text{ g} + 1.25 \text{ g} + 15 \text{ g} + 165.5 \text{ g} + 2 \text{ g} + 2 \text{ g} = 253.25 \text{ g}$$

(2) Total weight amount of the monomeric portion:

$$17.5 \text{ g} + (333.33 * 0.15) \text{ g} + 15 \text{ g} + 165.5 \text{ g} + 2 \text{ g} = 250 \text{ g}$$

(3) Remainder not being the monomeric portion:

$$253.25 \text{ g} - 250 \text{ g} = 3.25 \text{ g}$$

(4) Remainder expressed in percent of the total weight amount:

$$3.25 \text{ g} = 1.28 \text{ w } \%$$

(5) Total weight amount of feed 1 not including deonized water:

$$(333.33 * 0.15) \text{ g} + 1.25 \text{ g} + 15 \text{ g} + 165.5 \text{ g} + 2 \text{ g} + 2 \text{ g} = 235.75 \text{ g}$$

(6) 5 w% of total weight amount of water-depleted feed 1

$$11.7875 \text{ g} = 5 \text{ w } \% \text{ of } (5)$$

(7) Remainder not being the monomeric portion in (6):

$$11.7875 \text{ g} * 1.28 \text{ w } \% = 0.1509 \text{ g}$$

(8) Amount of aliquot I of the monomeric portion in water-depleted feed I:

$$11.7875 \text{ g} - 0.1509 \text{ g} = 11.6366 \text{ g}$$

(9) Amount of aliquot I:

$$11.6366 \text{ g} + 17.5 \text{ g} = 29.14 \text{ g}$$

(10) Aliquot I corresponds to a percent amount of the monomeric portion:

$$29.14 \text{ g} * (100/250 \text{ g}) = 11.66 \text{ w } \% \text{ of the monomeric portion}$$

The amount of aliquot S in the raw mixture is calculated as follows:

The raw mixture contains 5 w % of the amount of feed I, which includes. 5 w % of the total amount of colloidal silica sol used, which is 0.1 g.

The amount of aliquot P is calculated as shown below:

The raw mixture contains 5 w % of the amount of feed I, which includes 5 w % phosphoric acid used, which is 0.0625 g.

The amount of aliquot A is calculated as follows:

The raw mixture contains 5 w% of the amount of feed 2, which includes 5 w % of the total weight amount of Wako V 50 used, which is 0.1 g.

The amount of N-vinylpyrrolidone in the raw mixture is calculated as follows:

17.5 g + (165.5 g * 0.05) = 25.775 g = 14.09 w % of the total amount of N-vinylpyrrolidone in the monomeric portion.

Said raw mixture is heated under nitrogen up to 70 °C and supplemented with the remaining portion of feed 1 during 4 h and with the remaining portion of feed 2 during 4 h 30 min. The reaction mixture is then stirred for another two hours at 70 °C and thereafter heated at 80 °C and supplemented with feed 3.

Feed 3 comprises:

| | | |
|---|---|---|
| Tert-butylhydroperoxide (TBHP) | 1.25 g | aqueous solution containing 70 w % of TBHP |
| Deonized water | 45 g | |

After stirring for another 10 min feed 4 is added during 30 min.

Feed 4 comprises:

| | |
|---|---|
| Sodium disulfite | 1 g |
| Deonized water | 7.5 g |

Another 2 h of stirring is realized and thereafter a water vapor distillation at 120 °C after which the product obtained is cooled at 40 °C. Said product is a mostly highly viscous solution hardly to be stirred.

Table 2: Examples 2 to 7

| | Example 2 | Example 3 | Example 4 | Example 5 | Exampe 6 | Example 7 |
|---|---|---|---|---|---|---|
| | Amount [g] | | | | | |
| Raw Mixture | | | | | | |
| Deionized water | 362.00 | 362.00 | 362.00 | 362.00 | 362.00 | 362.00 |
| N-Vinyl-2-pyrrolidone | 17.50 | 17.50 | 17.50 | 17.50 | 17.50 | 17.50 |
| From Feed 1 | 26.00 | 26.00 | 25.85 | 25.75 | 25.85 | 25.86 |
| From Feed 2 | 3.00 | 3.00 | 2.73 | 2.73 | 2.73 | 3.00 |
| Feed 1 | | | | | | |
| Methacrylamide | 333.33 (6) | 333.33 (6) | 333.33 (6) | 333.33 (6) | 333.33 (6) | 333.33 (6) |
| Phosphoric acid | 1.25 (7) | 1.25 | 1.25 | 1.25 | 1.25 | 1.30 |
| 1-Vinyl-imidazol | 15.00 | 15.00 | 15.00 | 15.00 | 15.00 | 15.00 |
| N-Vinyl-2-pyrrolidone | 166.50 | 167.00 | 165.50 | 165.50 | 165.50 | 142.50 + 25 (10) |
| MEMO (1) | 1.00 | 0.50 | 2.00 | - | - | - |
| Colloidal Silica (2) | 2.00 | 2.00 | - | - | 2.00 | - |

(continued)

| | | | | | | |
|---|---|---|---|---|---|---|
| Feed 2 | | | | | | |
| Deionized water | 52.50 | 52.50 | 52.50 | 52.50 | 52.50 | 52.50 |
| Wako V 50 (8) | 2.00 | 2.00 | 2.00 | 2.00 | 2.00 | 1.25 |
| Feed 3 | | | | | | |
| t-Butylhydro-peroxide (9) | 1.25 | 1.25 | 1.25 | 1.25 | 1.25 | 1.25 |
| Deionized water | 45.00 | 45.00 | 45.00 | 45.00 | 45.00 | 7.50 |
| Feed 4 | | | | | | |
| Sodiium Disulfite | 1.00 | 1.00 | 1.00 | 1.00 | 1.00 | 1.00 |
| Deionized water | 7.50 | 7.50 | 7.50 | 7.50 | 7.50 | 45.00 |
| (1) = 3-methacryloyloxy)-propyl trimethoxysilane<br>(2) = Levasil 200 A<br>(6) = 15 w % solution<br>(7) = 85 w % solution<br>(8) = Trade name for 2,2'-azobis(2-methylpropionamidine)dihydrochloride<br>(9) = 70 w % solution<br>(10) = N-Vinyl-2-pyrrolidone + N-Vinyl-ε-caprolactam | | | | | | |

Examples 2 to 7 were conducted like example 1 using the different reagents in the indicated amounts.

By way of example different hair styling compositions, especially hair styling gels comprising the copolymers of the invention are shown below.

Hair styling gel 1:

Phase A

| | |
|---|---|
| 0,50 g | Carbomer or acrylates/C10-30 alkyl acrylate crosspolymer |
| 91,20 g | Deionized water |

Phase B

| | |
|---|---|
| 0,90 g | Tetrahydroxypropyl ethylenediamine |

Phase C

| | |
|---|---|
| 7,00 g | Copolymer of the invetion |
| 0,20 g | Parfume oil / volatile oils |
| q.s. | Preservative |
| 0,10 g | Propylene glycol |

| Har styling gel 2: | [%] |
|---|---|
| Phase A | |
| 0,50 g | Carbomer or acrylates/C10-30 alkyl acrylate crosspolymer |
| 86,40 g | Deionized water |
| Phase B | |
| 0,70 g | Triethanolamine |
| Phase C | |
| 11,00 g poly | Styling polymer or combination of e.g.: polyvinylpyrrolidone, vinylpyrrolidone, vinylacetate copolymer, polyquaternium-4 |
| (PQ-4), | |
| | PQ-11, PQ-16, PQ-46, PQ-44, PQ-68, vinylpyrrolidone /methacrylamide/ vinylimidazole copolymer, etc. |
| 0,20 g | Polyethyleneglycol-25-para-aminobenzoic acid |
| 2,00 g | Polyurethane PU.1 |
| 0,10 g | Parfume oil / volatile oil |
| q.s. | Polyethyleneglycol-14 dimethicone |
| q.s. | Preservative |
| 0,10 g | Tocopheryl Acetate |

One realized the invention to disclose a styling copolymer comprising colloidal silica, a styling composition of said copolymer a process for making it and its use for hair styling purposes. Said copolymer comprises colloidal silica and a copolymerized monomeric portion of at least one water-soluble monoolefinic monomer A and at least one monoolefinic silane monomer B with the weight portion of monomer B being less than 1 % of the total weight of the monomeric portion. The copolymerized monomeric portion in certain embodiments also comprises a monomer C.

## Claims

1. Copolymer for hair styling purposes comprising colloidal silica and a copolymerized monomeric portion of

   - at least one water-soluble monoolefinic monomer A, and
   - at least one monoolefinic silane monomer B

   wherein the weight portion of monomer B is less than 1 % of the total weight of the monomeric portion.

2. Copolymer according to claim 1, wh ere i n the monomeric portion comprises 30 to 99.9 w % of monomer A, preferably 60 to 99.2 w % and in a mostly preferred embodiment between 80 and 99.1 w %.

3. Copolymer according to claim 1 or 2, wherein the weight portion of monomer B ranges from 0.01 to 0.99 % of the total weight of the monomeric portion, preferably from 0.1 to 0.9 % and in a most preferred embodiment from 0.2 to 0.8 %.

4. Copolymer according to any one of claims 1 to 3, wherein monomer B is a vinylic reactive silane monomer.

5. Copolymer according to any one of claims 1 to 4, wherein monomer B has the general formula

   $$R^5\text{-}(CH_2)_n\text{-}Si(R^6)_2R^7$$

   with

   - $R^5$ = acryloyloxy-, methacryloyloxy-, vinyloxy-, vinyl-;
   - n = 0 to 10;
   - $R^6$ = -O-$(CH_2)_m$-$CH_3$ with m = 0 to 4, isopropyl-, isopropyloxy, tert-butyl-, tert-butyloxy-, sec-butyl-, sec-butyloxy;

- R7 = $R^6$, -H, $-CH_3$, $-CH_2CH_3$.

**6.** Copolymer according to any one of claims 1 to 5, wherein monomer B is (3-methacryloyloxy)-propyl trimethoxysilane (MEMO) and/or vinyltrimethoxysilane (VTMOS).

**7.** Copolymer according to any one of claims 1 to 6, wherein per 100 parts by weight of monomeric portion it comprises between 0.01 and 50 parts by weight, preferably between 0.1 and 30 parts by weight, and in a still more preferred embodiment between 0.2 and 20 parts by weight of colloidal silica.

**8.** Copolymer according to any one of claims 1 to 7, wherein the colloidal silica has a medium particle size of 5 to 200 nm, preferably of 5 to 75 nm and in a mostly preferred embodiment of 15 to 55 nm.

**9.** Copolymer according to any one of claims 1 to 8, wherein the colloidal silica has a specific surface area ranging from 50 to 500 $m^2/g$, more preferably from 100 to 350 $m^2/g$, even more preferably from 200 $m^2/g$ to 300 $m^2/g$ and most preferably being 200 $m^2/g$.

**10.** Copolymer according to any one of claims 1 to 9, wherein the colloidal silica has a morphological structure as found in a silica sol.

**11.** Copolymer according to any one of claims 1 to 10, wherein at least 10 w % of the colloidal silica, preferably 50 w % and more preferably 80 w % thereof is covalently bond to at least one monomer B of the copolymerized monomeric portion.

**12.** Copolymer according to any one of claims 1 to 11, wherein the colloidal silica is a silica sol and the monomer B is (3-methacryloyloxy)-propyl trimethoxysilane (MEMO) and/or vinyltrimethoxysilane (VTMOS).

**13.** Hair styling composition comprising a copolymer according to any one of claims 1 to 12, wherein it has a stickiness according to Kempf as disclosed in the present description at 20 °C and 80 relative humidity. ranging from 1 to 4, preferably from 2 to 4 and in a mostly preferred embodiment from 3 to 4.

**14.** Process for making a copolymer according to any one of claims 1 to 12, compriing

- placing water or an aqueous mixture in a vessel;
- charging the not yet polymerized monomeric portion or an aliquot II thereof into the vessel;
- adding at least one initiator to actuate copolymerization;

wherein the monomeric portion or the aliquot II thereof comprises

- at least one water-soluble monoolefinic monomer A,
- at least one monoolefinic silane monomer B,
- the weight portion of monomer B being less than 1 % of the total weight of the monomeric portion,
- and contacting said monomeric portion with colloidal silica prior and/or during copolymerization.

**15.** Process according to claim 14, wherein the water or the aqueous mixture contains at least one water-soluble monoolefinic monomer A.

**16.** Process according to claim 14 or 15, wherein the water or the aqueous mixture contains an aliquot I of the monomeric portion, said aliquot I making 4 to 25 w % of the total weight amount of the monomeric portion, preferably 6 to 18 w %, more preferably 8 to 14 w % and in a particularly preferred embodiment 10 to 12 w % thereof.

**17.** Process according to any one of claims 14 to 16, wherein the water or the aqueous mixture is heated up to 60 to 80 °C preferably up to 70 °C under an inert atmosphere.

**18.** Process according to any one of claims 14 to 17, wherein the monomeric portion or the aliquot II thereof is charged into the water or into the aqueous mixture in a period of time ranging from 3 h to 5 h, preferably 4 h.

**19.** Process according to any one of claims 14 to 18, wherein the initiator is used in an amount of 0.01 to 10 w %, preferably of 0.3 to 5 w % of the amount of the monomeric portion.

**20.** Process according to any one of claims 14 to 19, wherein the initiator is added at a vessel temperature ranging from

70 °C to 80 °C.

**21.** Process according to any one of claims 14 to 20, wherein the colloidal silica used is a silica sol.

**22.** Process according to any one of claims 14 to 21, wherein the monomeric portion and colloidal silica are supplied from a first inlet and the free radical initiator is added to the reaction vessel from at least one inlet different from the first inlet.

**23.** Process according to any one of claims 14 to 22, wherein the copolymerization is carried out at temperatures ranging from 50 °C to 220 °C, preferably from 50 °C to 100 °C.

**24.** Process according to any one of claims 14 to 23, wherein the copolymerization is realized under pressure at temperatures from 95 °C up to 220 °C.

**25.** Process according to any one of claims 14 to 24, wherein the copolymer is synthesized by radical-polymerization in solution.

**26.** Use of a copolymer according to any one of claims 1 to 25, comprising colloidal silica and a copolymerized monomeric portion of

- at least one water-soluble monoolefinic monomer A, and
- at least one monoolefinic silane monomer B

for cosmetic purposes particularly for hair styling purposes.

## Patentansprüche

**1.** Copolymer für Haarstylingzwecke, umfassend kolloidale Kieselsäure und einen copolymerisierten monomeren Teil aus

- mindestens einem wasserlöslichen monoolefinischen Monomer A und
- mindestens einem monoolefinischen Silanmonomer B,

wobei der Gewichtsanteil von Monomer B weniger als 1 Gew.-% des Gesamtgewichts des monomeren Teils beträgt.

**2.** Copolymer nach Anspruch 1, wobei der monomere Teil 30 bis 99,9 Gew.-% Monomer A, vorzugsweise 60 bis 99,2 Gew.-% und in einer ganz besonders bevorzugten Ausführungsform zwischen 80 und 99,1 Gew.-% umfasst.

**3.** Copolymer nach Anspruch 1 oder 2, wobei der Gewichtsanteil von Monomer B im Bereich von 0,01 bis 0,99% des Gesamtgewichts des monomeren Teils, vorzugsweise von 0,1 bis 0,9% und in einer ganz besonders bevorzugten Ausführungsform von 0,2 bis 0,8% beträgt.

**4.** Copolymer nach einem der Ansprüche 1 bis 3, wobei es sich bei Monomer B um ein vinylisches reaktives Silanmonomer handelt.

**5.** Copolymer nach einem der Ansprüche 1 bis 4 wobei das Monomer B die allgemeine Formel

$$R^5\text{-}(CH_2)_n\text{-}So(R^6)_2R^7$$

mit

- $R^5$ = Acryloyloxy-, Methacryloyloxy-, Vinyloxy-, Vinyl-;
- n = 0 bis 10;
- $R^6$ - $-O-(CH_2)_m-CH_3$ mit m = 0 bis 4, Isopropyl-, Isopropyloxy-, tert.-Butyl-, tert.-Butyloxy-, sec.-Butyl-, sec.-Butyloxy;
- $R^7$ = $R^6$, -H, $-CH_3$, $-CH_2CH_3$

aufweist.

6. Copolymer nach einem der Ansprüche 1 bis 5, wobei es sich bei Monomer B um (3-Methacryloyloxy)-propyltrimethoxysilan (MEMO) und/oder Vinyltrimethoxysilan (VTMOS) handelt.

7. Copolymer nach einem der Ansprüche 1 bis 6, wobei es pro 100 Gewichtsteile monomerem Teil zwischen 0,01 und 50 Gewichtsteile, vorzugsweise zwischen 0,1 und 30 Gewichtsteile und in einer noch weiter bevorzugten Ausführungsform zwischen 0,2 und 20 Gewichtsteile kolloidale Kieselsäure umfasst.

8. Copolymer nach einem der Ansprüche 1 bis 7, wobei die kolloidale Kieselsäure eine mittlere Teilchengröße von 5 bis 200 nm, vorzugsweise 5 bis 75 nm und in einer ganz besonders bevorzugten Ausführungsform 15 bis 55 nm aufweist.

9. Copolymer nach einem der Ansprüche 1 bis 8, wobei die kolloidale Kieselsäure eine spezifische Oberfläche im Bereich von 50 bis 500 $m^2/g$, weiter bevorzugt von 100 bis 350 $m^2/g$, noch weiter bevorzugt von 200 $m^2/g$ bis 300 $m^2/g$ und ganz besonders bevorzugt von 200 $m^2/g$ aufweist.

10. Copolymer nach einem der Ansprüche 1 bis 9, wobei die kolloidale Kieselsäure eine morphologische Struktur aufweist, wie sie in einem Kieselsäuresol anzutreffen ist.

11. Copolymer nach einem der Ansprüche 1 bis 10, wobei mindestens 10 Gew.-% der kolloidalen Kieselsäure, vorzugsweise 50 Gew.-% und weiter bevorzugt 80 Gew.-% davon kovalent an mindestens ein Monomer B des copolymerisierten monomeren Teils gebunden sind.

12. Copolymer nach einem der Ansprüche 1 bis 11, wobei es sich bei der kolloidalen Kieselsäure um ein Kieselsäuresol handelt und es sich bei Monomer B um (3-Methacryloyloxy)propyltrimethoxysilan (MEMO) und/oder Vinyltrimethoxysilan (VTMOS) handelt.

13. Haarstylingzusammensetzung, umfassend ein Copolymer nach einem der Ansprüche 1 bis 12, wobei sie eine wie in der vorliegenden Beschreibung offenbarte Klebrigkeit gemäß Kempf bei 20°C und einer relativen Feuchtigkeit von 80 im Bereich von 1 bis 4, vorzugsweise von 2 bis 4 und in einer ganz besonders bevorzugten Ausführungsform von 3 bis 4 aufweist.

14. Verfahren zur Herstellung eines Copolymers nach einem der Ansprüche 1 bis 12, bei dem man

- in einem Gefäß Wasser oder eine wässrige Mischung vorlegt;
- in das Gefäß den noch nicht polymerisierten monomeren Teil oder ein Aliquot II davon einträgt;
- mindestens einen Initiator zugibt, um die Copolymerisation in Gang zu bringen;

wobei der monomere Teil oder das Aliquot II davon

- mindestens ein wasserlösliches monoolefinisches Monomer A und
- mindestens ein monoolefinisches Silanmonomer B umfasst,
- wobei der Gewichtsanteil von Monomer B weniger als 1 Gew.-% des Gesamtgewichts des monomeren Teils beträgt,
- und den monomeren Teil vor und/oder während der Copolymerisation mit kolloidaler Kieselsäure in Berührung bringt.

15. Verfahren nach Anspruch 14, bei dem das Wasser bzw. die wässrige Mischung mindestens ein wasserlösliches monoolefinisches Monomer A enthält.

16. Verfahren nach Anspruch 14 oder 15, bei dem das Wasser bzw. die wässrige Mischung ein Aliquot I des monomeren Teils enthält, wobei das Aliquot I 4 bis 25 Gew.-% der gesamten Gewichtsmenge des monomeren Teils, vorzugsweise 6 bis 18 Gew.-%, weiter bevorzugt 8 bis 14 Gew.-% und in einer besonders bevorzugten Ausführungsform 10 bis 12 Gew.-% davon ausmacht.

17. Verfahren nach einem der Ansprüche 14 bis 16, bei dem man das Wasser bzw. die wässrige Mischung unter einer Inertatmosphäre auf 60 bis 80°C, vorzugsweise bis 70°C, erhitzt.

18. Verfahren nach einem der Ansprüche 14 bis 17, bei dem man den monomeren Teil bzw. das Aliquot II davon in einem Zeitraum im Bereich von 3 h bis 5 h, vorzugsweise 4 h, in das Wasser bzw. in die wässrige Mischung einträgt.

**19.** Verfahren nach einem der Ansprüche 14 bis 18, bei dem man den Initiator in einer Menge von 0,01 bis 10 Gew.-%, vorzugsweise 0,3 bis 5 Gew.-%, der Menge des monomeren Teils verwendet.

**20.** Verfahren nach einem der Ansprüche 14 bis 19, bei dem man den Initiator bei einer Gefäßtemperatur im Bereich von 70°C bis 80°C zugibt.

**21.** Verfahren nach einem der Ansprüche 14 bis 20, bei dem es sich bei der verwendeten kolloidalen Kieselsäure um ein Kieselsäuresol handelt.

**22.** Verfahren nach einem der Ansprüche 14 bis 21, bei dem man den monomeren Teil und die kolloidale Kieselsäure aus einem ersten Einlass zuführt und den radikalischen Initiator aus mindestens einem von dem ersten Einlass verschiedenen Einlass in das Reaktionsgefäß gibt.

**23.** Verfahren nach einem der Ansprüche 14 bis 22, bei dem man die Copolymerisation bei Temperaturen im Bereich von 50°C bis 220°C, vorzugsweise von 50°C bis 100°C, durchführt.

**24.** Verfahren nach einem der Ansprüche 14 bis 23, bei dem man die Copolymerisation unter Druck bei Temperaturen von 95°C bis 220°C durchführt.

**25.** Verfahren nach einem der Ansprüche 14 bis 24, bei dem man das Copolymer durch radikalische Polymerisation in Lösung herstellt.

**26.** Verwendung eines Copolymers nach einem der Ansprüche 1 bis 25, umfassend kolloidale Kieselsäure und einen copolymerisierten monomeren Teil aus

- mindestens einem wasserlöslichen monoolefinischen Monomer A und
- mindestens einem monoolefinischen Silanmonomer B,

für kosmetische Zwecke, insbesondere für Haarstylingzwecke.

## Revendications

**1.** Copolymère à des fins de coiffage comprenant de la silice colloïdale et une partie monomère copolymérisée constituée de

- au moins un monomère monooléfinique hydrosoluble A et
- au moins un monomère silane monooléfinique B,

dans lequel la proportion en poids de monomère B est inférieure à 1 % du poids total de la partie monomère.

**2.** Copolymère selon la revendication 1, dans lequel la partie monomère comprend 30 à 99,9 % en poids de monomère A, de préférence 60 à 99,2 % en poids et dans un mode de réalisation particulièrement préféré entre 80 et 99,1 % en poids.

**3.** Copolymère selon la revendication 1 ou 2, dans lequel la proportion en poids de monomère B va de 0,01 à 0,99 % du poids total de la partie monomère, de préférence de 0,1 à 0,9 % et dans un mode de réalisation préféré pardessus tout de 0,2 à 0,8 %.

**4.** Copolymère selon l'une quelconque des revendications 1 à 3, dans lequel le monomère B est un monomère silane réactif vinylique.

**5.** Copolymère selon l'une quelconque des revendications 1 à 4, dans lequel le monomère B répond à la formule générale

$$R^5\text{-}(CH^2)_n\text{-}Si(R^6)_2R^7$$

avec

- $R^5$ = acryloyloxy-, méthacryloyloxy-, vinyloxy-, vinyl- ;
- n = 0 à 10 ;
- $R^6$ - $-O-(CH_2)_m-CH_3$ avec m = 0 à 4, isopropyl-, isopropyloxy-, tert-butyl-, tert-butyloxy-, sec-butyl-, sec-butyloxy- ;
- $R^7 = R^6$, -H, $-CH_3$, $-CH_2CH_3$.

**6.** Copolymère selon l'une quelconque des revendications 1 à 5, dans lequel le monomère B est le (3-méthacryloyloxy) propyltriméthoxysilane (MEMO) et/ou le vinyltriméthoxysilane (VTMOS).

**7.** Copolymère selon l'une quelconque des revendications 1 à 6, dans lequel pour 100 parties en poids de partie monomère il y a entre 0,01 et 50 parties en poids, de préférence entre 0,1 et 30 parties en poids et dans un mode de réalisation encore davantage préféré entre 0,2 et 20 parties en poids de silice colloïdale.

**8.** Copolymère selon l'une quelconque des revendications 1 à 7, dans lequel la silice colloïdale a une taille moyenne des particules de 5 à 200 nm, de préférence de 5 à 75 nm et dans un mode de réalisation particulièrement préféré de 15 à 55 nm.

**9.** Copolymère selon l'une quelconque des revendications 1 à 8, dans lequel la silice colloïdale a une surface spécifique allant de 50 à 500 $m^2/g$, plus préférablement de 100 à 350 $m^2/g$, encore plus préférablement de 200 $m^2/g$ à 300 $m^2/g$ et de préférence par-dessus tout qui est de 200 $m^2/g$.

**10.** Copolymère selon l'une quelconque des revendications 1 à 9, dans lequel la silice colloïdale a une structure morphologique telle que trouvée dans un sol de silice.

**11.** Copolymère selon l'une quelconque des revendications 1 à 10, dans lequel au moins 10 % en poids de la silice colloïdale, de préférence 50 % en poids et plus préférablement 80 % en poids de celle-ci est liée de façon covalente à au moins un monomère B de la partie monomère copolymérisée.

**12.** Copolymère selon l'une quelconque des revendications 1 à 11, dans lequel la silice colloïdale est un sol de silice et le monomère B est le (3-méthacryloyloxy)propyltriméthoxysilane (MEMO) et/ou le vinyltriméthoxysilane (VTMOS).

**13.** Composition de coiffage comprenant un copolymère selon l'une quelconque des revendications 1 à 12, laquelle a un caractère collant selon Kempf tel qu'exposé dans la présente description à 20 °C et une humidité relative de 80 % allant de 1 à 4, de préférence de 2 à 4 et dans un mode de réalisation particulièrement préféré de 3 à 4.

**14.** Procédé pour la fabrication d'un copolymère selon l'une quelconque des revendications 1 à 12, comprenant

- l'introduction d'eau ou d'un mélange aqueux dans une cuve ;
- le chargement de la partie monomère pas encore polymérisée ou d'une aliquote II de celle-ci dans la cuve ;
- l'ajout d'au moins un initiateur pour amorcer la copolymérisation ;

la partie monomère ou l'aliquote II de celle-ci comprenant

- au moins un monomère monooléfinique hydrosoluble A,
- au moins un monomère silane monooléfinique B,

la proportion en poids de monomère B étant inférieure à 1 % du poids total de la partie monomère,

- et la mise en contact de ladite partie monomère avec de la silice colloïdale avant et/ou pendant la copolymérisation.

**15.** Procédé selon la revendication 14, dans lequel l'eau ou le mélange aqueux contient au moins un monomère monooléfinique hydrosoluble A.

**16.** Procédé selon la revendication 14 ou 15, dans lequel l'eau ou le mélange aqueux contient une aliquote I de la partie monomère, ladite aliquote I constituant 4 à 25 % en poids de la quantité totale en poids de la partie monomère, de préférence 6 à 18 % en poids, plus préférablement 8 à 14 % en poids et dans un mode de réalisation particulièrement préféré 10 à 12 % en poids de celle-ci.

**17.** Procédé selon 1"une quelconque des revendications 14 à 16, dans lequel l'eau ou le mélange aqueux est chauffé jusqu'à 60 à 80 °C de préférence jusqu'à 70 °C sous une atmosphère inerte.

**18.** Procédé selon l'une quelconque des revendications 14 à 17, dans lequel la partie monomère ou l'aliquote II de celle-ci est chargée dans l'eau ou dans le mélange aqueux sur une durée allant de 3 h à 5 h, de préférence de 4 h.

**19.** Procédé selon l'une quelconque des revendications 14 à 18, dans lequel l'initiateur est utilisé en une quantité de 0,01 à 10 % en poids, de préférence de 0,3 à 5 % en poids de la quantité de la partie monomère.

**20.** Procédé selon l'une quelconque des revendications 14 à 19, dans lequel initiateur est ajouté à une température de cuve allant de 70 °C à 80 °C.

**21.** Procédé selon l'une quelconque des revendications 14 à 20, dans lequel la silice colloïdale utilisée est un sol de silice.

**22.** Procédé selon l'une quelconque des revendications 14 à 21, dans lequel la partie monomère et la silice colloïdale sont introduites à partir d'une première entrée et l'initiateur de radicaux libres est ajouté au réacteur à partir d'au moins une entrée différente de la première entrée.

**23.** Procédé selon l'une quelconque des revendications 14 à 22, dans lequel la copolymérisation est effectuée à des températures allant de 50 °C à 220 °C, de préférence de 50 °C à 100 °C.

**24.** Procédé selon l'une quelconque des revendications 14 à 23, dans lequel la copolymérisation est réalisée sous pression à des températures allant de 95 °C jusqu'à 220 0 °C.

**25.** Procédé selon l'une quelconque des revendications 14 à 24, dans lequel le copolymère est synthétisé par polymérisation radicalaire en solution.

**26.** Utilisation d'un copolymère selon l'une quelconque des revendications 1 à 25, comprenant de la silice colloïdale et une partie monomère copolymérisée constituée de

- au moins un monomère monooléfinique hydrosoluble A et
- au moins un monomère silane monooléfinique B, à des fins cosmétiques en particulier à des fins de coiffage.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005082322 A1


### Non-patent literature cited in the description


- **FIKENTSCHER.** *Cellulosechemie,* 1932, vol. 13, 58-64